(19) Europäisches Patentamt
European Patent Office
Office européen des brevets

(11) Numéro de publication : **0 520 880 A1**

(12) **DEMANDE DE BREVET EUROPEEN**

(21) Numéro de dépôt : **92401765.0**

(22) Date de dépôt : **24.06.92**

(51) Int. Cl.$^5$ : **C07D 513/04,** A61K 31/54, // (C07D513/04, 279:00, 205:00)

(30) Priorité : **25.06.91 FR 9107785**

(43) Date de publication de la demande : **30.12.92 Bulletin 92/53**

(84) Etats contractants désignés : **AT BE CH DE DK ES FR GB GR IT LI LU NL PT SE**

(71) Demandeur : **ROUSSEL-UCLAF**
**35, Boulevard des Invalides**
**F-75007 Paris (FR)**

(72) Inventeur : **Chantot, Jean-François**
**2, Allée Eole**
**F-77410 Gressy en France (FR)**
Inventeur : **Gouin D'Ambrières, Solange**
**17, rue Lis Franc**
**F-75020 Paris (FR)**
Inventeur : **Humbert, Daniel**
**15, rue Gaston Charle**
**F-94120 Fontenay sous Bois (FR)**
Inventeur : **Teutsch, Jean-Georges**
**Résidence Lavoisier, Bât. 3, 3, rue Lavoisier**
**F-93500 Pantin (FR)**

(74) Mandataire : **Bourgouin, André et al**
**Département des Brevets ROUSSEL UCLAF**
**111, route de Noisy B.P. no 9**
**F-93230 Romainville (FR)**

(54) **Nouvelles céphalosporines comportant un noyau 2-thia céphème, leur procédé de préparation, leur application comme médicaments, les compositions pharmaceutiques les renfermant et les nouveaux intermédiaires obtenus.**

(57) L'invention a pour objet les produits de formule générale (I) :

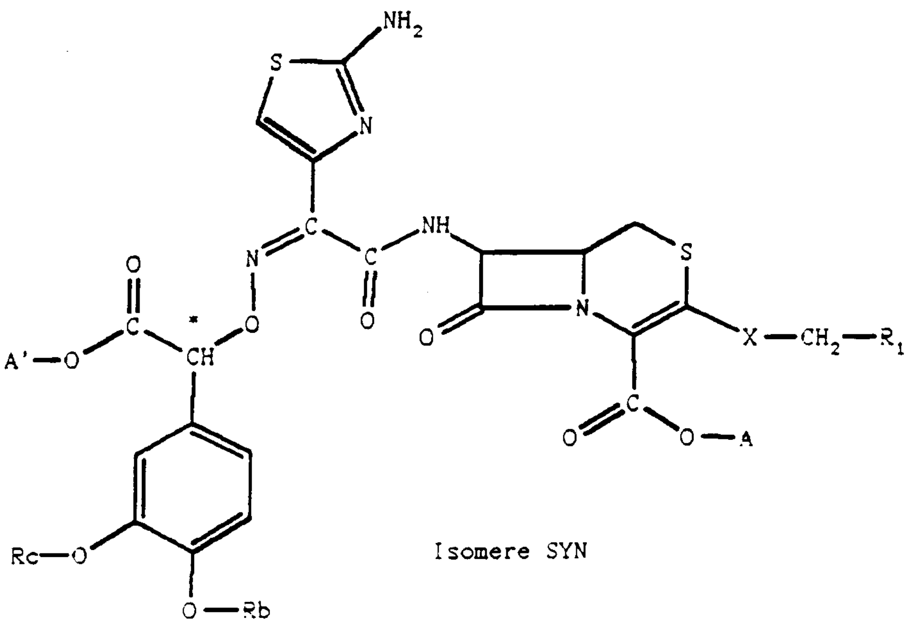

(I)

Isomere SYN

isomère syn, sous forme R ou S ou d'un mélange R, S, formule dans laquelle :
$R_1$ représente soit un radical hétérocyclique,
soit un radical -S-Het,
X représente une simple liaison ou un radical C = C sous forme E ou Z,
$R_b$ et $R_c$, représentent un atome d'hydrogène ou un groupement acyle,
A et A′ représentent un atome d'hydrogène, un équivalent de base, ou le reste d'un groupement ester

EP 0 520 880 A1

facilement clivable ou $CO_2A$ représente $CO_2^{\ominus}$ ainsi que les sels des produits de formule (I) avec les acides minéraux ou organiques.

Ces produits présentent d'intéressantes propriétés qui justifient leur utilisation en antibiothérapie.

La présente invention concerne de nouvelles céphalosporines comportant un noyau 2-thia céphème, leur procédé de préparation, leur application comme médicaments, les compositions pharmaceutiques les renfermant et les nouveaux intermédiaires obtenus.

L'invention a pour objet les produits de formule générale (I) :

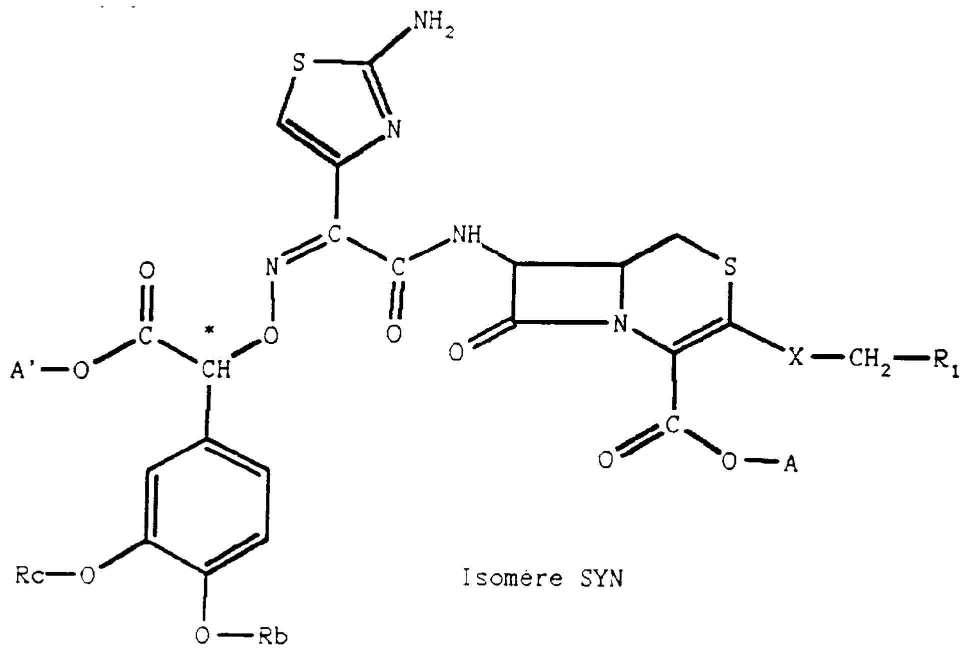

(I)

Isomère SYN

isomère syn, sous forme R ou S ou d'un mélange R, S, formule dans laquelle :

$R_1$ représente soit un radical choisi parmi les radicaux suivants :

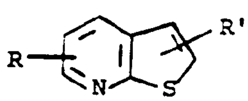
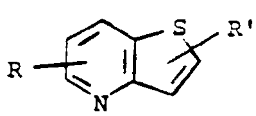
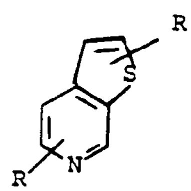
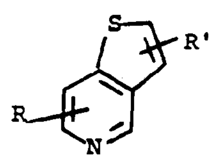

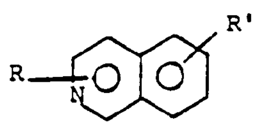
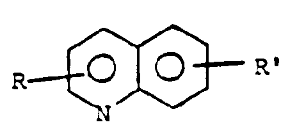
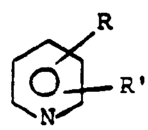
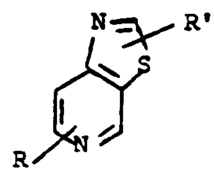

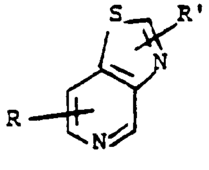
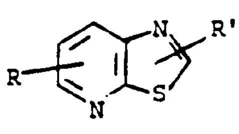
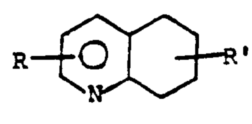
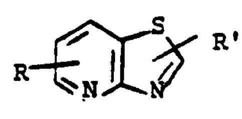

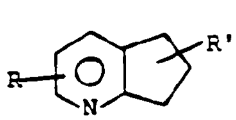
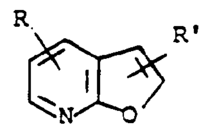
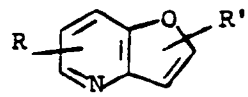
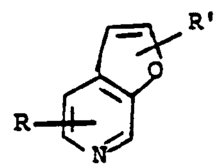

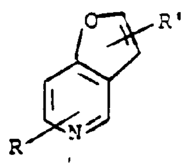
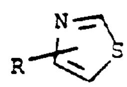
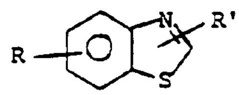
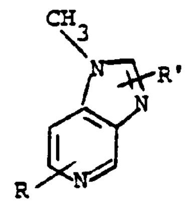
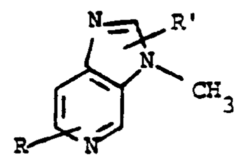

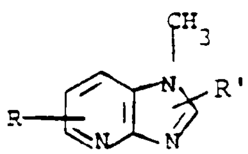
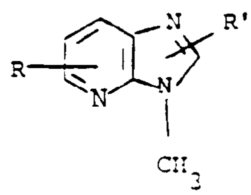
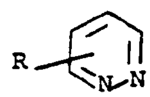
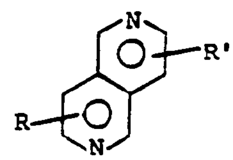

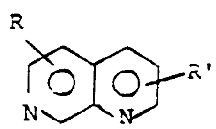
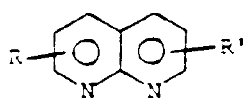
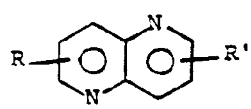
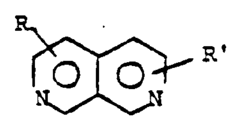

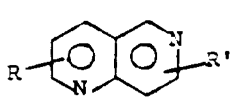
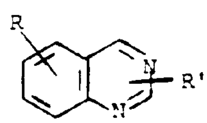
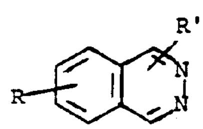
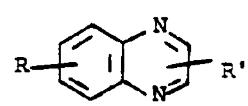

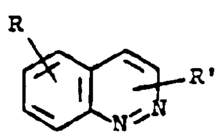
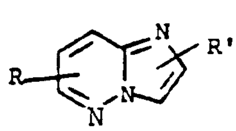
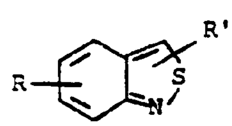
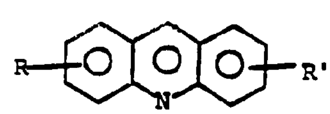

ou

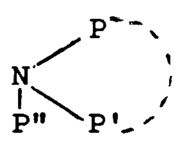 ,

sous forme d'ammonium quaternaire, ou

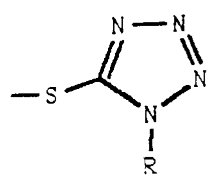 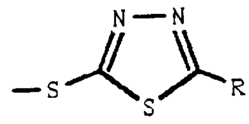

dans lesquels R et R′ identiques ou différents représentent un atome d'hydrogène, un radical alkyle renfermant de 1 à 4 atomes de carbone, un radical alcoxy renfermant de 1 à 4 atomes de carbone, un atome d'halogène, un radical $CO_2$-Q,

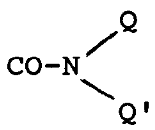 , 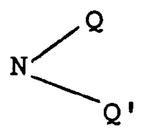 , 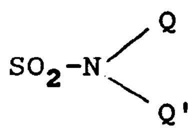 , 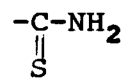 ,

NH-CO-Q, CN, $CH_2$-CN, $CH_2$-SQ dans lesquels Q et Q′ identiques ou différents représentent un atome d'hydrogène ou un radical alkyle renfermant de 1 à 4 atomes de carbone, P, P′ et P″ identiques ou différents représentent un radical alkyle renfermant au plus 4 atomes de carbone, éventuellement substitué par un des substituants indiqués ci-dessus pour R et R′, le trait pointillé indiquant que P et P′ peuvent éventuellement former avec l'atome d'azote auquel ils sont liés, un hétérocycle à 5 ou 6 chaînons,

soit $R_1$ représente un radical -S-Het dans lequel Het représente un hétérocycle choisi parmi les radicaux thiazolyle, isothiazolyle, oxazolyle, isoxazolyle, triazolyle, thiadiazolyle, oxadiazolyle, tétrazolyle, imidazolyle, pyrazolyle, pyridinyle, pyrimidinyle, pyrazinyle, pyridazinyle, triazinyle, dihydrotriazinyle, ces radicaux étant éventuellement substitués par un ou plusieurs substituants choisis parmi les radicaux alkyle ayant de 1 à 4 atomes de carbone, de préférence méthyle, amino, alkyl ou dialkylamino hydroxy, oxo, alkylthio, carboxy ou carboxyalkyle libres, estérifiés ou salifiés.

X représente une simple liaison ou un radical $>C = C<$ sous forme E ou Z.

$R_b$ et $R_c$, identiques ou différents représentent un atome d'hydrogène ou un groupement acyle,

A et A′ identiques ou différents représentent un atome d'hydrogène, un équivalent de métal alcalin, alcalino-terreux, de magnésium, d'ammonium ou d'une base organique aminée ou A et A′ représentent le reste d'un groupement ester facilement clivable ou $CO_2A$ représente $CO_2^{\ominus}$ ainsi que les sels des produits de formule (I) avec les acides minéraux ou organiques.

Lorsque $R_b$ et/ou $R_c$ représentent un radical acyle, il peut s'agir des radicaux acétyle, propionyle, benzoyle ou formyle. La valeur acyle préférée est la valeur acétyle. La valeur préférée pour $R_b$ et $R_c$ est la valeur hydrogène.

Parmi les valeurs de A et de A′ on peut citer un équivalent de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

On peut citer entre autres restes de groupements esters facilement clivables que peuvent représenter A et A′, les groupements méthoxyméthyle, éthoxyméthyle, isopropyloxyméthyle, alpha-méthoxy éthyle, alpha-éthoxy éthyle, méthylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butylcarbonyloxyméthyle, hexadécanoyloxyméthyle, propionyloxyéthyle, isovaléryloxyéthyle, 1-acétyloxyéthyle, 1-propionyloxyéthyle, 1-butyryloxyéthyle, 1-tert-butylcarbonyloxyéthyle, 1-acétyloxypropyle, 1-hexadécanoyloxyéthyle, 1-propionyloxypropyle, 1-méthoxycarbonyloxyéthyle, méthoxycarbonyloxyméthyle, 1-acétyloxybutyle, 1-acétyloxyhexyle, 1-acétyloxyheptyle, phtalidyle, 5,6-diméthoxyphtalidyle, tert-butylcarbonylméthyle, allyle, 2-chloroallyle, méthoxycarbonylméthyle, benzyle, tert-butyle, trityle, benzhydryle ou para-méthoxybenzyle.

On peut encore citer entre autres restes de groupements esters que peuvent représenter A et A′, les groupements méthoxyéthoxyméthyle, diméthylaminoéthyle, cyanométhyle, tert-butoxycarbonylméthyle, 2,2-éthylènedioxyéthyle, cyanoéthyle, 2,2-diméthoxyéthyle ; 2-chloroéthoxyméthyle, 2-hydroxyéthoxyéthyle, 2,3-époxypropyle, 3-diméthylamino, 2-hydroxypropyle, 2-hydroxyéthyle, 2-méthylaminoéthoxyméthyle, 2-aminoéthoxyméthyle, 3-méthoxy 2,4-thiadiazol-5-yle, 2-tétrahydropyrannyle, 1-méthoxy 1-méthyl éthyle, 2-hydroxy 1-méthyl éthyle, isopropyle ; carbamoylméthyle, chlorométhyle, 2-chloroéthyle, acétylméthyle, 2-méthylthioéthyle ou thiocyanatométhyle.

On peut encore citer entre autres restes de groupements esters que peuvent représenter A et A′, les groupements 2-chloro 1-acétyloxyéthyle, 2-bromo 1-acétyloxyéthyle, 2-fluoro 1-acétyloxyéthyle, 2-méthoxy 1-acétyloxyéthyle, 2-méthyl 1-acétyloxypropyle, 1-méthyl 1-acétyloxyéthyle, 1-méthoxyacétyloxyéthyle, 1-acétylcarbonyloxyéthyle, 1-hydroxyacétyloxyéthyle, 1-formylcarbonyloxyéthyle, 1-(2-thiényl) carbonyloxyéthyle, 1-(2-furyl) carbonyloxyéthyle, 1-(5-nitro 2-furyl) carbonyloxyéthyle, 1-(2-pyrrolyl) carbonyloxyéthyle, 1-(propionyloxycarbonyloxy) éthyle, 1-(propyloxycarbonyloxy) éthyle, 1-(isopropyloxycarbonyloxy) éthyle, 1-(méthoxyéthoxycarbonyloxy) éthyle, 1-(allyloxycarbonyloxy) éthyle, isopropyloxycarbonyl méthyle, 1-[(2,3-époxy propyl) oxycarbonyloxy] éthyle, 1-[(2-furyl) méthyloxycarbonyloxy] éthyle, 1-(2-fluoro éthyl) oxycarbonyloxyéthyle, 1-(méthoxycarbonyloxy) propyle, 1-(méthoxycarbonyloxy) 1-méthyl éthyle, (méthoxycarbonyloxy) chlorométhyle, 1-(méthoxycarbonyloxy) 2-chloroéthyle, 1-(méthoxycarbonyloxy) 2-méthoxy éthyle, 1-(méthoxycarbonyloxy) allyle, ou un reste :

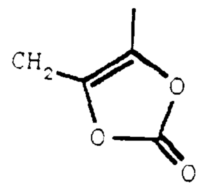

Par valeur alkyle ayant de 1 à 4 atomes de carbone, on entend les valeurs méthyle, éthyle, propyle, butyle, isobutyle, sec-butyle et tert-butyle. On préfère en général la valeur méthyle.

Par alkoxy ayant de 1 à 4 atomes de carbone, on entend les valeurs formées avec les radicaux alkyle indiqués ci-dessus par exemple, les valeurs méthoxy, éthoxy, propyloxy, butyloxy. On préfère méthoxy en général.

Les atomes d'halogène sont choisis parmi les atomes de fluor, de chlore, de brome et d'iode. On préfère les atomes de fluor, de chlore ou de brome.

L'hétérocycle à 5 ou 6 chaînons que P et P′ peuvent former avec l'atome d'azote auquel ils sont liés peut être choisi parmi les radicaux pipéridine, morpholine, pyrrolidine.

Parmi les valeurs alkylamino que peuvent porter les hétérocycles que représente Het, on peut citer les radicaux méthylamino, éthylamino, isopropylamino, butyl (linéaire ou ramifié) amino. Le radical alkyle comporte donc de 1 à 4 atomes de carbone.

Il en est de même des radicaux alkyle que peut porter le radical dialkylamino. Parmi les radicaux dialkylamino on peut citer les radicaux diméthylamino, diéthylamino, méthyléthylamino par exemple.

Parmi les valeurs alkylthio, on préfère les radicaux dans lesquels le radical alkyle comporte de 1 à 4 atomes de carbone, tels que méthyle, éthyle, propyle, butyle ou butyle ramifié. On préfère le radical méthylthio.

Les radicaux carboxyalkyle peuvent être formés avec les mêmes radicaux alkyles que ceux indiqués ci-dessus. On préfère le radical carboxyméthyle. Ces radicaux carboxy et carboxyalkyle peuvent être estérifiés ou salifiés comme indiqué ci-dessus.

Parmi les radicaux hétérocycliques que peut représenter Het, on peut citer les radicaux 1,3,4, 1,2,4 ou 1,2,3 thiadiazolyle ; thiazol-2-yle, imidazol-2-yle ; 2-méthyl 1,3,4-thiadiazol-5-yle ; 1-méthyl tétrazol-5-yle ; les dihydrotriazinyles de formules suivantes :

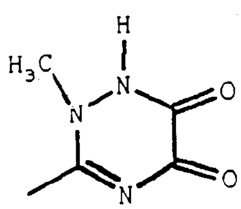
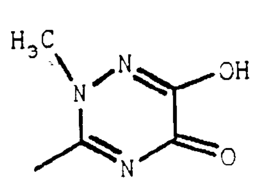
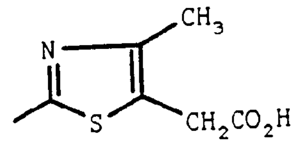
;

le noyau isocéphème peut être sous forme racémique ou optiquement active, on préfère les produits dans lesquels ce noyau est (S) cis c'est-à-dire (S);(S).

Les produits de formule (I) peuvent également se présenter sous forme de sels d'acides organiques ou minéraux.

Parmi les acides avec lesquels on peut salifier le ou les groupements amino des produits (I), on peut citer entre autres, les acides acétique, trifluoroacétique, maléïque, tartrique, méthanesulfonique, benzènesulfonique, para-toluène sulfonique, phosphorique, sulfurique, chlorhydrique, bromhydrique, iodhydrique.

Les produits peuvent également se présenter sous forme de sels internes.

Dans un mode préféré de l'invention, A' représente un atome d'hydrogène ou de sodium, de préférence hydrogène et $CO_2A$ représente $CO_2^{\ominus}$.

L'invention a plus particulièrement pour objet les produits de formule générale (I) telle que définie ci-dessus dans laquelle $R_1$ est choisi

soit parmi les radicaux :

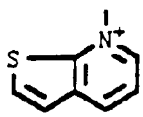
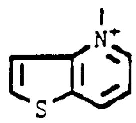
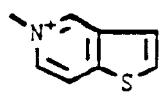
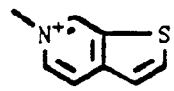
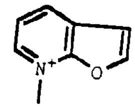
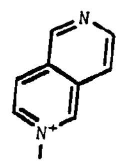

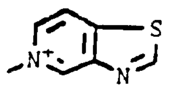
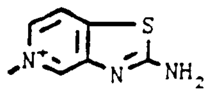
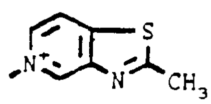
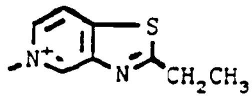
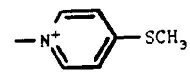

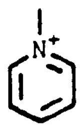
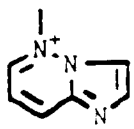
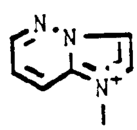
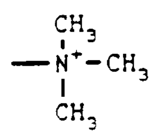
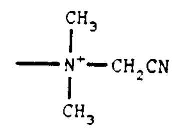

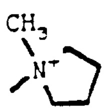
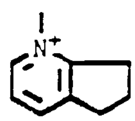
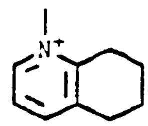
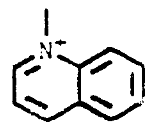
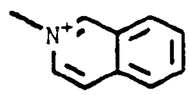
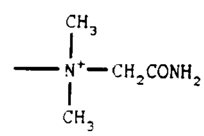

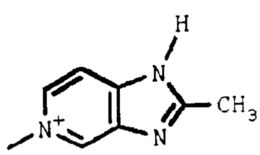
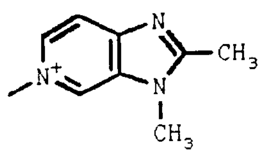
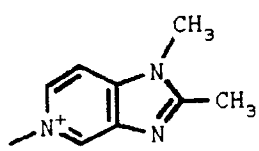
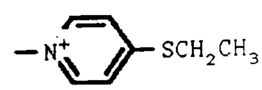

soit parmi les radicaux S-Het, dans lesquels Het représente les radicaux 1,2,4, 1,3,4 ou 1,2,3-thiadiazolyle ; thiazole-2-yle ; imidazol-2-yle ; 2-méthyl 1,3,4-thiadiazol-5-yle ; 1-méthyl tétrazol-5-yle et les radicaux :

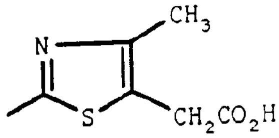
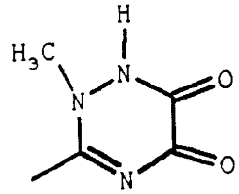
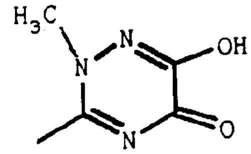

L'invention a plus particulièrement pour objet les produits de formule générale (I) telle que définie ci-dessus dans laquelle $R_1$ est choisi

soit parmi les radicaux :

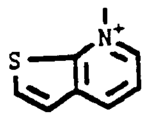 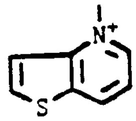 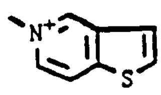 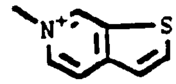

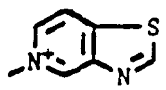 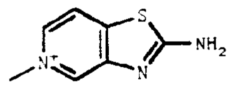 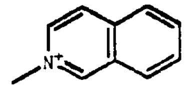

de préférence le radical :

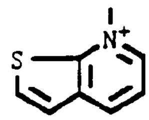

soit $R_1$ représente un radical 1,3,4-thiadiazolylthio ou méthyltétrazolylthio.

L'invention a particulièrement pour objet les produits définis ci-après dans les exemples, et plus particulièrement - le (S)(cis)(Z) 5-[[7-[[(2-amino 4-thiazolyl) [[carboxy-(3,4-dihydroxy phényl) méthoxy] imino] acétyl] amino] 2-carboxy 8-oxo 4-thia 1-azabicyclo[4,2,0]oct-2-èn-3-yl] méthyl] thiéno[2,3-b]pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables.

Il est entendu que les produits de formule (I) précités peuvent exister :

soit sous la forme indiquée par ladite formule (I),

soit sous la forme de produits de formule $(I)_Z$ :

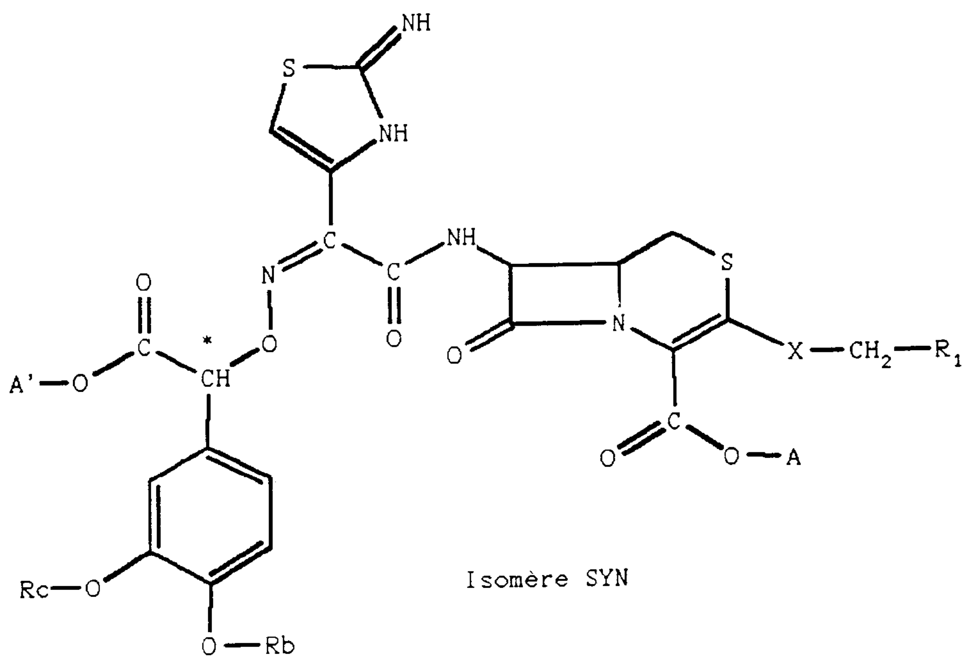

$(I)_Z$

Isomère SYN

dans laquelle A, A', X, Rb, Rc et $R_1$ ont la signification indiquée précédemment.

L'invention a également pour objet un procédé de préparation des produits de formule (I) telle que définie ci-dessus, caractérisé en ce que

soit l'on fait agir un produit de formule (II) :

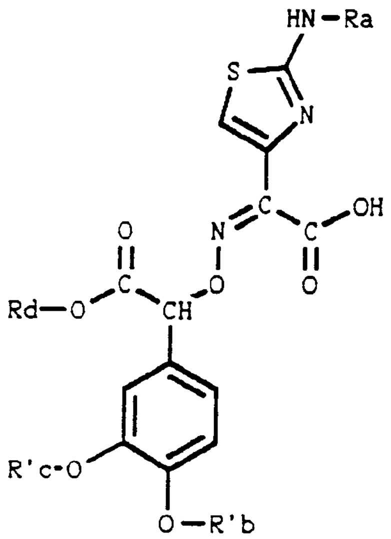

(II)

isomère syn, racémique ou optiquement actif ou un dérivé fonctionnel du produit de formule (II), dans laquelle $R_a$ représente un atome d'hydrogène ou un groupement protecteur du radical amino, $R'_b$ et $R'_c$ identiques ou différents représentent un atome d'hydrogène ou un groupement protecteur du radical hydroxyle, $R_d$ représente un atome d'hydrogène ou le reste d'un groupement ester facilement éliminable, avec un produit de formule (III) :

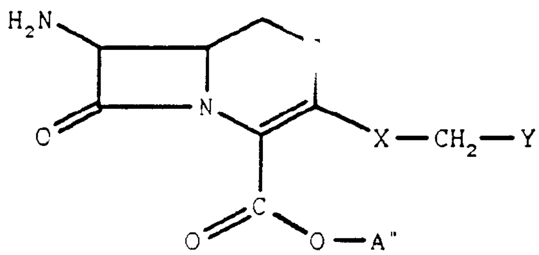

(III)

dans laquelle Y représente un radical hydroxy ou un atome d'halogène, A" représente un atome d'hydrogène ou le reste d'un groupement ester facilement éliminable pour obtenir un produit de formule (IV) :

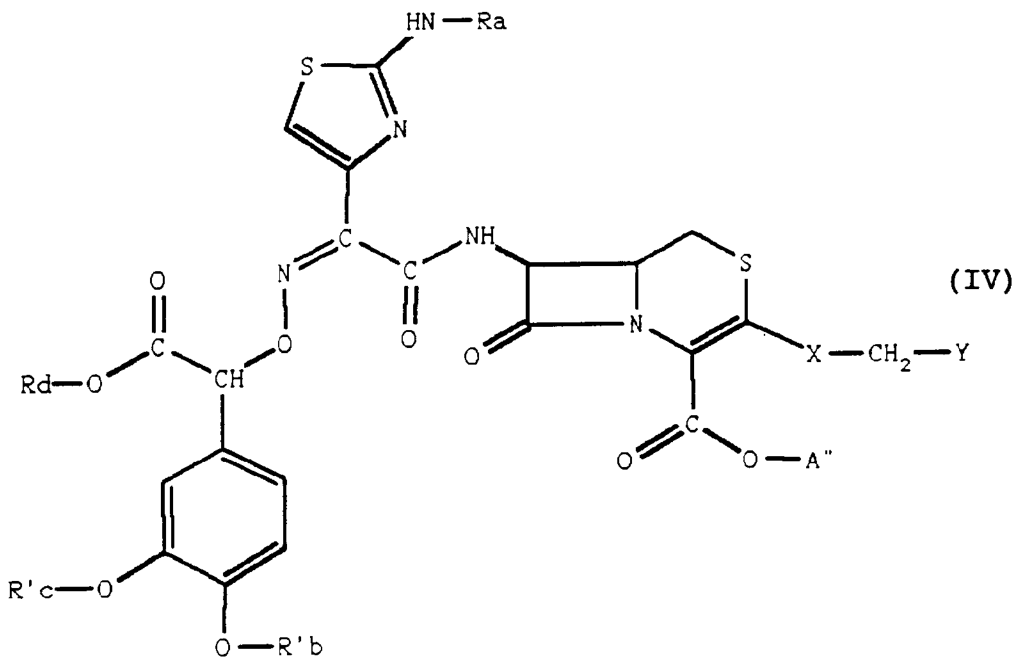

(IV)

que l'on fait réagir avec un réactif capable d'introduire le radical $R_1$ pour obtenir un produit de formule (V) :

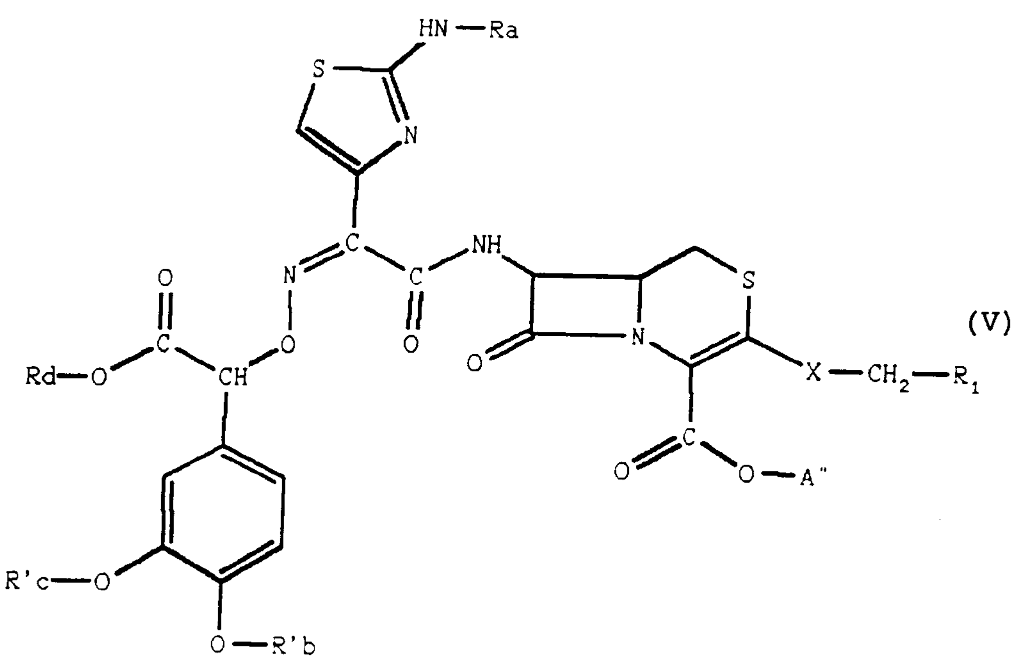

(V)

soit l'on fait réagir un produit de formule (VI) :

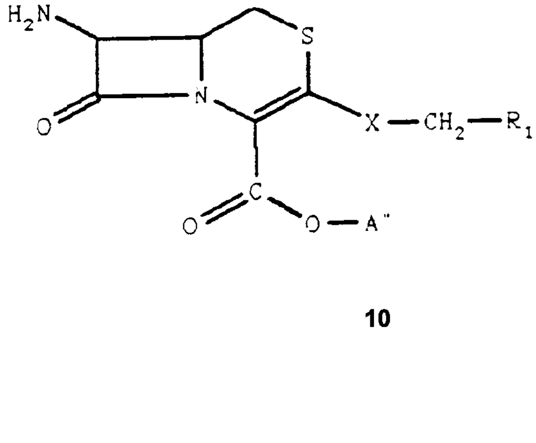

(VI)

dans laquelle X, $R_1$ et A″ ont la signification précédente avec un produit de formule (II) telle que définie ci-dessus ou un dérivé fonctionnel du produit de formule (II) pour obtenir un produit de formule (V) telle que définie ci-dessus,

produit de formule (V) que, si désiré, lorsque X représente un radical >C = C< l'on sépare en ses isomères E ou Z ou transforme les isomères Z en isomères E et produits de formule (V) que, si nécessaire ou si désiré, l'on soumet à une ou plusieurs des réactions suivantes, dans un ordre quelconque :

a) coupure par hydrolyse ou par action de la thiourée de tout ou partie des groupements esters ou des groupements de protection du radical amino ou des radicaux hydroxyles,

b) estérification ou salification par une base du ou des radicaux carboxyliques,

c) salification par un acide du radical amino,

d) séparation des produits sous forme de mélange R,S en R ou S.

En plus des groupements cités ci-dessus, les groupements ester facilement éliminable que peuvent représenter A″ et $R_d$ peuvent être par exemple l'ester formé avec les radicaux butyle, isobutyle, tert-butyle, pentyle, hexyle, acétoxyméthyle, propionyloxyméthyle, butyryloxyméthyle, valéryloxyméthyle, pivaloyloxyméthyle, 2-acétoxyéthyle, 2-propionyloxyéthyle, 2-butyryloxyéthyle.

On peut également citer les radicaux 2-iodoéthyle, 2,2,2-trichloro éthyle, vinyle, allyle, éthynyle, propynyle, benzyle, 4-méthoxybenzyle, 4-nitrobenzyle, phényléthyle, trityle, diphénylméthyle, 3,4-diméthoxyphényle.

On peut également citer les radicaux phényle, 4-chloro phényle, tolyle, tert-butylphényle.

On préfère le radical diphénylméthyle pour A″ et $R_d$.

Le groupement protecteur du radical amino que peut représenter $R_a$ peut être par exemple un radical alkyle de 1 à 6 atomes de carbone tel que, préférentiellement, tert-butyle ou tert-amyle. $R_a$ peut également représenter un groupement acyle, aliphatique, aromatique ou hétérocyclique ou un groupe carbamoyle. On peut citer les groupements alcanoyles inférieurs tel que par exemple formyle, acétyle, propionyle, butyryle, isobutyryle, valéryle, isovaléryle, oxalyle, succinyle, pivaloyle.

$R_a$ peut également représenter un groupe alkoxy ou cycloalkoxycarbonyle inférieur tel que par exemple, méthoxycarbonyle, éthoxycarbonyle, propoxycarbonyle, 1-cyclopropyléthoxycarbonyle, isopropyloxycarbonyle, butyloxycarbonyle, tert-butyloxycarbonyle, pentyloxycarbonyle, hexyloxycarbonyle, un groupe benzoyle, toluolyle, naphtoyle, phtaloyle, mésyle, phénylacétyle, phénylpropionyle, un groupe aralcoxycarbonyle, tel que benzyloxycarbonyle.

Les groupements acyles peuvent être substitués par exemple par un atome de chlore, de brome, d'iode ou de fluor. On peut citer les radicaux chloroacétyle, dichloroacétyle, trichloroacétyle, bromoacétyle ou trifluoroacétyle.

$R_a$ peut également représenter un groupement aralkyle inférieur tel que benzyle, 4-méthoxybenzyle, phényléthyle, trityle, 3,4-diméthoxybenzyle ou benzhydryle.

$R_a$ peut également représenter un groupe haloalkyle tel que trichloroéthyle.

$R_a$ peut également représenter un groupement chlorobenzoyle, para-nitrobenzoyle, para-tert-butylbenzoyle, phénoxyacétyle, caprylyle, n-décanoyle, acryloyle, trichloro-éthoxycarbonyle.

$R_a$ peut également représenter un groupement méthyl carbamoyle, phénylcarbamoyle, naphtylcarbamoyle, ainsi que les thiocarbamoyles correspondants.

On préfère le groupement trityle.

La liste ci-dessus n'est pas limitative, il est évident que d'autres groupements protecteurs des amines, groupements connus en particulier dans la chimie des peptides, peuvent également être utilisés.

Les groupements de protection des radicaux hydroxyles que peuvent représenter $R'_b$ et $R'_c$, peuvent être choisis dans la liste ci-dessous :

$R'_b$ et $R'_c$ peuvent représenter un groupe acyle tel que par exemple formyle, acétyle, propionyle, chloroacétyle, bromoacétyle, dichloro-acétyle, trichloroacétyle, trifluoroacétyle, méthoxyacétyle, phénoxyacétyle, benzoyle, benzoylformyle, p-nitrobenzoyle. On peut citer également les groupements éthoxy carbonyle, méthoxycarbonyle, propoxycarbonyle, 2,2,2-trichloro-éthoxycarbonyle, benzyloxycarbonyle, tert-butoxycarbonyle, 1-cyclopropyléthoxycarbonyle, tétrahydropyrannyle, tétrahydrothiopyrannyle, méthoxytétrahydropyrannyle, trityle, benzyle, 4-méthoxybenzyle, benzhydryle, trichloroéthyle, 1-éthyl 1-méthoxyéthyle, phtaloyle.

On peut également citer d'autres acyles tels que butyryle, isobutyryle, valéryle, isovaléryle, oxalyle, succinyle et pivaloyle.

On peut également citer les radicaux phénylacétyle, phénylpropionyle, mésyle, chlorobenzoyle, para-nitrobenzoyle, para-tert-butylbenzoyle, caprylyle, acryloyle, méthylcarbamoyle, phénylcarbamoyle, naphtylcarbamoyle.

On peut également citer les radicaux alkoxy alkoxy méthyle tel que méthoxy éthoxy méthyle.

Les radicaux $OR'_b$ et $OR'_c$ peuvent également former avec le radical phényle auquel ils sont liés, les valeurs suivantes :

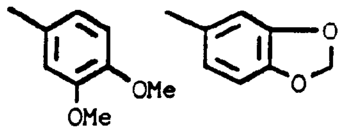 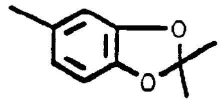 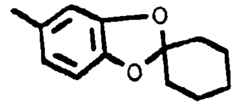

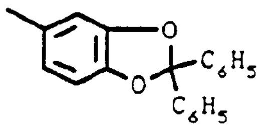 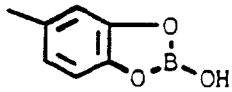 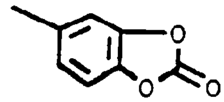

On préfère le groupement méthoxy éthoxy méthyle pour les substituants $R'_b$ et $R'_c$.

Dans un mode d'exécution du procédé, on fait agir un dérivé fonctionnel du produit de formule (II). Ce dérivé fonctionnel peut être par exemple un halogénure, un anhydride symétrique ou mixte, l'amide, l'azide ou un ester activé.

Comme exemple d'anhydride mixte on peut citer par exemple celui formé avec le chloroformiate d'isobutyle et celui formé avec le chlorure de pivaloyle et les anhydrides mixtes carboxylique-sulfonique formé par exemple avec le chlorure de paratoluène sulfonyle.

Comme exemple d'ester activé, on peut mentionner l'ester formé avec le 2,4-dinitrophénol et celui formé avec l'hydroxy-benzothiazole.

Comme exemple d'halogénure, on peut citer le chlorure ou le bromure.

L'anhydride peut être formé in situ par action de carbodiimide N,N'-disubstitué, par exemple le N,N-dicyclohexylcarbodiimide, le diisopropyl carbodiimide ou l'éthyldiméthylamino propyl carbodiimide.

La réaction d'acylation est conduite de préférence dans un solvant organique tel que le chlorure de méthylène. On peut cependant utiliser d'autres solvants tels que le tétrahydrofuranne, le chloroforme, l'acétone, l'eau ou le diméthylformamide.

Lorsqu'on utilise un halogénure d'acide et de manière générale lorsqu'une molécule d'acide halohydrique est libérée au cours de la réaction, on réalise la réaction de préférence en présence d'une base telle que la soude, la potasse, les carbonates et carbonates acides de sodium ou de potassium, l'acétate de sodium, la triéthylamine, la pyridine, la morpholine ou la N-méthylmorpholine.

La température de réaction est en général inférieure ou égale à la température ambiante.

On peut également faire agir directement un produit de formule (II) avec un produit de formule (III) en présence d'un carbodiimide telle que le diisopropylcarbodiimide. Un exemple d'une telle préparation est donnée plus loin dans la partie expérimentale.

Le réactif capable d'introduire le radical $R_1$ est soit un des radicaux indiqués ci-dessus, qui se présentent ensuite, après introduction, sous forme d'un ammonium quaternaire,
soit un réactif de formule Het-SH dans laquelle Het a la définition indiquée ci-dessus.

L'addition de ces réactifs sur le produit de formule (IV) est effectuée dans les conditions suivantes :

Lorsque Y représente par exemple un atome de chlore, on peut effectuer in situ ou séparément une substitution de l'atome de chlore par un atome d'iode en présence d'iodure de sodium puis ajoute ensuite le réactif désiré. Un exemple d'une telle réaction est décrit ci-après dans la partie expérimentale.

On peut également faire agir sur le produit de formule (IV) dans lequel Y représente un atome de chlore, le réactif désiré en présence de tétrafluoroborate d'argent.

Lorsque Y représente un radical hydroxy, on peut également transformer ce produit en produit dans lequel Y représente un atome d'iode par exemple par action d'iodure de tétrabutyl ammonium en présence de 2,6-lutidine et d'anhydride méthylsulfonique.

On peut également faire agir l'un des réactifs indiqués ci-dessus sur un produit de formule (IV) dans lequel le radical hydroxy a été transformé en radical réactif par exemple à l'aide d'un anhydride sulfonique tel que l'anhydride méthylsulfonique, paratoluène sulfonique ou de préférence, trifluorométhane sulfonique.

L'action des produits de formule Het-SH est effectuée dans des conditions semblables à celles indiquées ci-dessus. On peut notamment tranformer les produits de formule (IV) dans laquelle Y représente un radical hydroxy en dérivé réactif à l'aide d'un dérivé d'acide sulfonique tel que l'anhydride de l'acide trifluorométhanesulfonique.

On opère de préférence en présence d'une base qui peut par exemple être choisie parmi la 2,6-lutidine, la 2,4,6-collidine, la tétraméthylguanidine, le diazabicyclononène ou le diazabicyclo undécène.

On peut également opérer en présence d'un agent alkylant par exemple l'anhydride trifluorméthylsulfonique.

L'action des produits de formule (II) sur les produits de formule (VI) est effectuée dans les mêmes conditions que l'action des produits de formule (II) sur les produits de formule (III).

L'isomérie des produits de formule (V) peut être différente de celle des produits de formule (IV) utilisés au départ. Dans le cas où l'on isole l'isomère Z, on peut transformer cet isomère en isomère E selon les méthodes usuelles, notamment par action de l'iode.

Selon les valeurs de $R_a$, $R'_b$, $R'_c$, $R_d$ et A″, les produits de formule (V) peuvent ou non constituer des produits de formule (I).

Les produits de formule (V) constituent des produits de formule (I) lorsque $R_a$ représente un atome d'hydrogène, lorsque $R'_b$ et $R'_c$ ne représentent pas un groupement protecteur du radical hydroxyle que l'on désire éliminer, c'est-à-dire lorsque $R'_b$ et/ou $R'_c$ représentent un radical acyle et lorsque $R_d$ et A″ ne représentent pas, parmi les groupements esters facilement clivables, l'un de ceux que l'on désirerait éliminer.

Dans les autres cas, l'action sur le produit de formule (V) d'un ou plusieurs agents d'hydrolyse, d'hydrogénolyse ou de la thiourée a pour but d'éliminer le radical $R_a$ lorsque celui-ci représente un radical protecteur du radical amino, d'éliminer les radicaux $R'_b$ et $R'_c$ lorsque ceux-ci représentent un groupement protecteur des radicaux hydroxyle et/ou d'éliminer les radicaux $R_d$ et A″ lorsque ceux-ci représentent, parmi les groupements esters facilement clivables l'un de ceux que l'on désire éliminer.

Cependant, il est bien entendu possible d'éliminer $R_a$, $R'_b$ et $R'_c$ sans toucher aux substituants $R_d$ et A″ lorsque ceux-ci doivent être conservés. Il en est ainsi par exemple lorsque A″ représente un groupement ester que l'on souhaite conserver tel qu'un groupement propionyloxyméthyle.

La nature des réactifs à mettre en jeu dans un tel cas est bien connu de l'homme de métier. Des exemples de telles réactions sont donnés plus loin dans la partie expérimentale.

On trouvera par exemple une description des différentes méthodes d'élimination des différents groupements protecteurs dans la demande de brevet français B.F. 2.499.995.

Etant donné les groupements protecteurs préférés que l'on utilise : trityle pour $R_a$, méthoxy éthoxy méthyle pour $R'_b$ et $R'_c$, et diphénylméthyle pour $R_d$ et A″, on utilise de préférence l'acide trifluoroacétique sans solvant ou dans un solvant tel que l'anisole ou un mélange de solvants tels que anisole/chlorure de méthylène. On obtient alors un sel avec l'acide trifluoroacétique. On peut alors revenir à la base libre par action d'une base telle que le carbonate de triéthylamine.

La salification des produits peut être effectuée selon les méthodes usuelles.

La salification peut, par exemple, être obtenue par action sur un produit sous forme acide ou sur un solvat, par exemple, le solvat éthanolique ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate trisodique. On peut également faire appel à des sels d'acides organiques.

Comme sels d'acides organiques, on peut mentionner, par exemple, les sels de sodium d'acides carboxyliques aliphatiques, linéaires ou ramifiés, saturés ou insaturés de 1 à 18 et de préférence de 2 à 10 atomes de carbone. Les chaînes aliphatiques de ces acides peuvent être interrompues par un ou plusieurs hétéroatomes tels que l'oxygène ou le soufre ou substituées par des radicaux aryle, comme, par exemple : phényle, thiényle, furyle, par un ou plusieurs radicaux hydroxyles ou par un ou plusieurs atomes d'halogène tels que fluor, chlore ou brome, préférentiellement chlore, par un ou plusieurs radicaux carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques, des acides aromatiques suffisamment solubles comme, par exemple, des acides benzoïques substitués, de préférence par des radicaux alkyles inférieurs.

Comme exemples de tels acides organiques, on peut mentionner :
les acides formique, acétique, acrylique, butyrique, adipique, isobutyrique, n-caproïque, isocaproïque, chloropropioniques, crotonique, phénylacétique, 2-thiénylacétique, 3-thiénylacétique, 4-éthylphénylacétique, glutarique, l'ester monoéthylique de l'acide adipique, les acides hexanoïque, heptanoïque, décanoïque, oléïque, stéarique, palmitique, 3-hydroxy propionique, 3-méthoxy propionique, 3-méthylthio butyrique, 4-chloro butyrique, 4-phénylbutyrique, 3-phénoxy butyrique, 4-éthyl benzoïque, 1-propyl benzoïque.

On utilise cependant de préférence comme sels de sodium, l'acétate de sodium, le 2-éthyl hexanoate de sodium ou le diéthyl acétate de sodium.

La salification peut également être obtenue par action d'une base organique comme la triéthylamine, la diéthylamine, la triméthylamine, la propylamine, la N,N-diméthyl éthanolamine, le tris (hydroxyméthyl) amino méthane, la méthylamine, l'éthanolamine, la pyridine, la picoline, la dicyclohexyl amine, la morpholine et la benzylamine.

Elle peut également être obtenue par action de l'arginine, de la lysine, de la procaïne, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectuée dans les conditions usuelles.

L'estérification éventuelle des produits est effectuée dans les conditions classiques. On opère en général en faisant réagir l'acide de formule (I) ou un dérivé fonctionnel avec un dérivé de formule :

Z - Re

dans laquelle Z représente un radical hydroxyle ou un atome d'halogène tel que le chlore, le brome, l'iode et Re désigne le groupement ester à introduire, groupement dont une liste non exhaustive figure ci-dessus. Dans certains cas, il peut être avantageux d'effectuer une estérification sur un produit dont l'amine et/ou les groupements réactionnels présents sur l'oxyimino sont bloqués avant d'enlever le groupement protecteur de l'amine et du groupement réactionnel présents sur l'oxyimino.

Les produits de formule (I) comportent plusieurs carbones asymétriques. Dans le noyau cephème, qui comporte deux carbones asymétriques, les deux carbones sont de préférence dans la configuration S. Par ailleurs le radical présent sur la fonction oxyimino comporte également un carbone asymétrique :

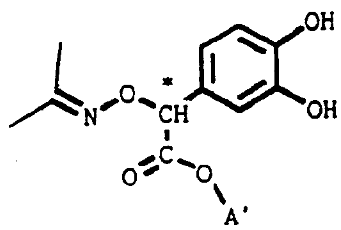

qui peut être sous forme R ou S ou sous forme d'un mélange R,S. La séparation des deux diastéréoisomères peut être effectuée par les moyens connus de l'homme du métier, par exemple par chromatographie.

Cette séparation peut être effectuée ou bien sur les produits de formule (I) ou bien sur les produits de formule (II).

Les produits de formule générale (I) possèdent une très bonne activité antibiotique sur les bactéries gram (+) telles que les staphylocoques, les streptocoques et notamment sur les staphylocoques pénicillino-résistants. Leur efficacité sur les bactéries gram (-) notamment sur les bactéries coliformes, les klebsiella, les salmonella, les proteus et les pseudomonas, est particulièrement remarquable.

Ces propriétés rendent aptes lesdits produits ainsi que leurs sels d'acides, pharmaceutiquement acceptables à être utilisés comme médicaments dans le traitement des affections à germes sensibles et notamment dans celui des staphylococies, telles que septicémies à staphylocoques, staphylococcies malignes de la face ou cutanée, pyodermites, plaies septiques ou suppurantes, anthrax, phlegmons, érésipèles, staphylococcies aiguës primitives ou post grippales, broncho-pneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

La présente invention a donc également pour objet, à titre de médicaments et notamment de médicaments antibiotiques, les produits de formule (I) tels que définis ci-dessus ainsi que leurs sels d'acides pharmaceutiquement acceptables.

L'invention a plus particulièrement pour objet à titre de médicaments les produits de formule (I) telle que décrite ci-dessus dans laquelle $R_1$ est choisi

soit parmi les radicaux :

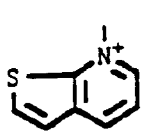 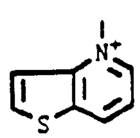 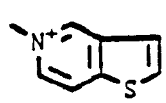 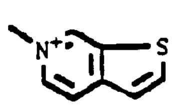 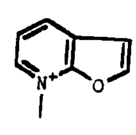 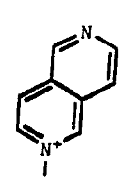

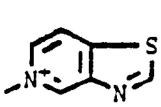 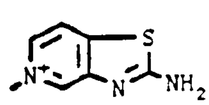 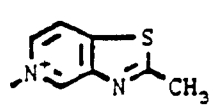 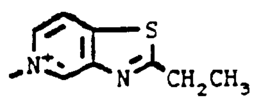 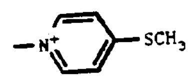

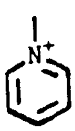 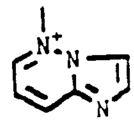 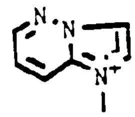 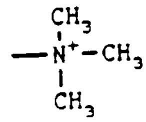 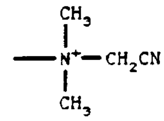

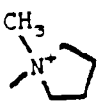 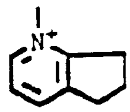 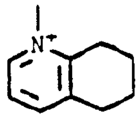 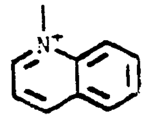 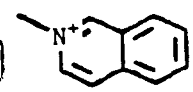 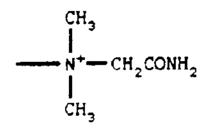

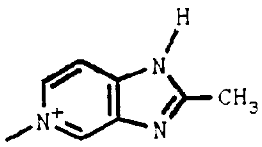 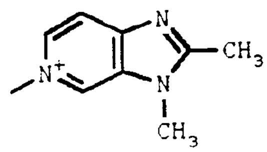 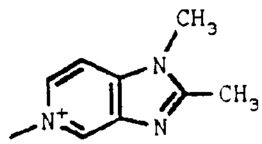 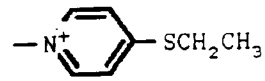

soit parmi les radicaux S-Het, dans lesquels Het représente les radicaux 1,2,4, 1,3,4 ou 1,2,3-thiadiazolyle ; thiazole-2-yle ; imidazol-2-yle ; 2-méthyl 1,3,4-thiadiazol-5-yle ; 1-méthyl tétrazol-5-yle et les radicaux :

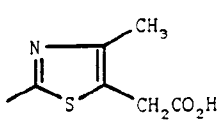 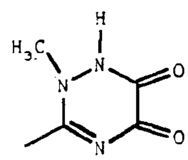 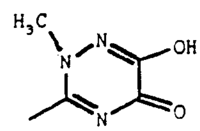

et plus particulièrement ceux dans laquelle $R_1$ est choisi
soit parmi les radicaux :

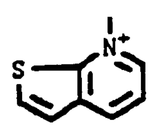 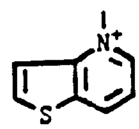 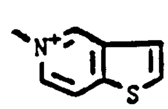 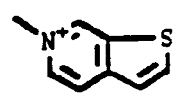

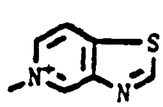 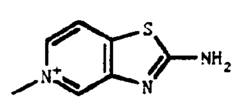 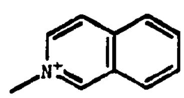

de préférence le radical :

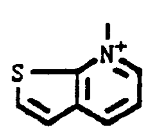

soit $R_1$ représente un radical 1,3,4-thiadiazolylthio ou méthyltétrazolylthio.

L'invention a spécialement pour objet, à titre de médicaments et notamment de médicaments antibiotiques, les produits décrits ci-après dans les exemples, et plus spécialement :
le (S)(cis)(Z) 5-[[7-[[(2-amino 4-thiazolyl) [[carboxy- (3,4-dihydroxy phényl) méthoxy] imino] acétyl] amino] 2-carboxy 8-oxo 4-thia 1-azabicyclo[4,2,0]oct-2-èn-3-yl] méthyl] thiéno[2,3-b]pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables.

L'invention s'étend aux compositions pharmaceutiques renfermant, comme principe actif, au moins un des médicaments définis ci-dessus.

Ces compositions peuvent être administrées par voie buccale, rectale, parentérale, notamment intramusculaire ou par voie locale en application topique sur la peau et les muqueuses.

Les produits de formule (I) et notamment ceux dans laquelle A représente un ester clivable peuvent être administrés par voie orale.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'une poudre destinée à être dissoute extemporanément dans un véhicule approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 4 g par jour, par voie orale chez l'homme, avec le produit décrit à l'exemple 1 ou encore comprise entre 0,500 g et 1 g trois fois par jour par voie intramusculaire.

Les produits de formule (I) peuvent également être utilisés comme désinfectants des instruments chirurgicaux.

L'invention a enfin pour objet, à titre de produits industriels nouveaux et notamment à titre de produits intermédiaires nécessaires à la préparation des produits de formule (I) telle que définie ci-dessus, les produits de formule (IV) et les produits de formule (V) dans laquelle $R_a$ représente un groupement protecteur du radical amino, les formules (IV) et (V) étant telles que définies ci-dessus.

Les produits de formule (II) sont connus dans la littérature, notamment dans les demandes de brevets européens EP 0.238.061 ou EP 0.266.060 ou peuvent être préparés selon les méthodes usuelles.

Les produits de formules (III) et (VI) sont également connus dans la littérature, notamment dans les demandes de brevets européens 153.229, 214.029, 282.365.

Les exemples suivants illustrent l'invention sous toutefois la limiter.

**<u>EXEMPLE 1</u> : (±)(cis)(Z) iodure de 2-amino-5-[[7-[[[2-amino 4-thiazolyl] [[carboxy (3,4-dihydroxy phényl) méthoxy] imino] acétyl amino] 2-carboxy 8-oxo 4-thia 1-aza bicyclo[4,2,0]oct-2-èn-3-yl] méthyl] thiazolo[4,5-c]pyridinium bis(trifluoroacétate)**

<u>STADE A</u> : [6S-[6alpha,7béta(Z)]] 7-[[[[(3,4-dihydroxy phényl) [(diphénylméthoxy) carbonyl] méthoxy] imino] [2-[(triphénylméthyl) amino] 4-thiazolyl] acétyl] amino] 3-(chlorométhyl) 8-oxo 4-thia 1-aza bicyclo[4,2,0]oct-2-ène-2-carboxylate de 1,1-diméthyléthyle

On agite pendant 18 heures un mélange de 600 mg d'acide [[1-(3,4-dihydroxy phényl) 2-(diphénylméthoxy) 2-oxo éthoxy] imino] [2-[(triphénylméthyl) amino] thiazol-4-yl] acétique, avec 115 mg d'hydroxybenzotriazole à 13 % d'eau, 240 mg de 3-chlorométhyl 7-amino 8-oxo 4-thia 1-aza bicyclo[4,2,0]oct-2-èn-2-carboxylate de 1,1-diméthyléthyle, avec 6 $cm^3$ de tétrahydrofuranne et 560 microlitres de diisopropylcarbodiimide. On évapore le solvant et chromatographie le résidu sur silice, éluant chlorure de méthylène-méthanol (98-2). On obtient 513 mg du produit recherché.

| Spectre de RMN | |
|---|---|
| OtBu | 1,57 (s) |
| $\underline{CH_2}Cl$ | 4,38 (d) 4,53 (d) 4,68 (d) 4,72 (d) |
| $-S-\underline{CH_2}-$ | 2,60 à 3,10 (m) |
| $-S-CH_2-\underline{CH}$ | 3,88 à 4,10 (m) |
| N | |
| $-C-C-\underline{NH}-$ | 8,35 (d) 8,01 (d) |
| O | |
| H | |
| $>\underline{C}-NH$ | 5,46 (d) 5,90 (d) |
| les protons aromatiques | 6,58 à 7,40 (m) |

STADE B : [6S-[6alpha,7béta(Z)]] 7-[[[[(3,4-dihydroxy phényl) [(diphénylméthoxy) carbonyl] méthoxy] imino] [2-[(triphénylméthyl) amino] 4-thiazolyl] acétyl] amino] 3-(iodométhyl) 8-oxo 4-thia 1-aza bicyclo[4,2,0]oct-2-ène-2-carboxylate de 1,1-diméthyléthyle

On agite pendant 2 heures à 40°C, 247 mg du produit obtenu ci-dessus, 5 $cm^3$ d'acétonitrile et 54 mg d'iodure de sodium. On refroidit, filtre le chlorure de sodium formé et évapore le solvant. Le produit est utilisé tel quel pour le stade suivant.

STADE C : iodure de (±) [6S-[6alpha,7béta(Z)]] 2-amino 5-[[7-[[[[(3,4-dihydroxy phényl) [(diphénylméthoxy) carbonyl] méthoxy] imino] [2-[(triphénylméthyl) amino] 4-thiazolyl] acétyl] amino] 2-[(11-diméthyléthoxy) carbonyl] 8-oxo 4-thia 1-aza bicyclo[4,2,0]oct-2-èn-3-yl] méthyl] thiazolo[4,5-c]pyridinium

On agite 18 heures à 20°C un mélange composé du produit obtenu ci-dessus avec 55 mg d'aminothiazolopyridine et 1 $cm^3$ de diméthyl sulfoxyde. On évapore en partie le solvant et chromatographie sur silice (éluant chlorure de méthylène seul puis chlorure de méthylène-acétone (90-10) et enfin chlorure de méthylène-méthanol (95-5)) on obtient 124 mg de produit recherché.

| Spectre de RMN : DMSO 300 MHz ppm | |
|---|---|
| OtBu | 1,48 - 1,49 (s) |
| CH-NH | 5,40 à 5,70 (m) |
| Protons noyau pyridine | 8,95 (s) 9,02 (s) 8,45 à 8,60 (m) |
| $-S-\underline{CH_2}-$ | 2,40 à 3,10 (m) |
| $-S-CH_2-\underline{CH}$ | 3,95 à 4,05 (m) |
| $-C(=O)-NH-\underline{CH}<$ | 5,40 à 5,70 (m) |
| $S-\underline{CH}$ | 6,59 à 6,82 (m) |

STADE D : (±)(cis)(Z) iodure de 2-amino-5-[[7-[[[2-amino 4-thiazolyl] [[carboxy (3,4-dihydroxy phényl) méthoxy] imino] acétyl amino] 2-carboxy 8-oxo 4-thia 1-aza bicyclo[4,2,0]oct-2-èn-3-yl] méthyl] thiazolo[4,5-c] pyridinium bis(trifluoroacétate)

On agite pendant 2 heures à 20°C, 115 mg du produit obtenu au stade précédent avec 0,5 $cm^3$ d'acide trifluoroacétique à 10 % d'anisole. On ajoute 5 $cm^3$ d'éther, agite 10 minutes, essore, lave à l'éther et sèche sous pression réduite. On obtient 71 mg du produit recherché.

<u>Spectre de RMN</u> (DMSO 300 MHz) ppm

| | |
|---|---|
| S-<u>$CH_2$</u>- | 2,80 à 2,40 |
| $S-CH_2$-<u>CH</u>-CH | 3,98 à 4,04 (m) |
| $-S-CH_2$-CH-<u>CH</u> | 5,64 (m) |
| -O-<u>CH</u>-$CO_2H$ | 5,23 (s) 5,31 (s) |
| les aromatiques | 6,6 à 6,8 |
| -S-<u>CH</u> | 6,6 à 6,8 |
| NH | 9,17 |
| <u>$CH_2$</u>-$N^{\ominus}$ | 5,44 (d) 5,78 (d) |

**<u>EXEMPLE 2</u> : (±)(cis)(Z) acide 7-[[[2-amino 4-thiazolyl] [[carboxy (3,4-dihydroxy phényl) méthoxy] imino] acétyl amino] 8-oxo 3-[3-[(1,3,4-thiadiazol 2-yl) thio] 1-(E)-propényl 4-thia 1-aza bicyclo[4,2,0]oct-2-ène-2-carboxylique (trifluoroacétate)**

<u>STADE A</u> : [6S-[3(E)6alpha,7béta(Z)]] 7-[[[[(3,4-dihydroxy phényl) [(diphénylméthoxy) carbonyl] méthoxy] imino] [2-[(triphénylméthyl) amino] 4-thiazolyl] acétyl] amino] 3-[3-[(1,3,4-thiadiazol-2-yl) thio] 1-propényl] 8-oxo 4-thia 1-aza bicyclo[4,2,0]oct-2-ène-2-carboxylate de 1,1-diméthyléthyle

On agite 20 heures à 20°C, 260 mg de 3-[3-[(1,3,4-thiadiazol-2-yl) thio] 1-propényl 7-amino 8-oxo 4-thia 1-aza bicyclo[4,2,0]oct-2-ène-2-carboxylate de 1,1-diméthyléthyle avec 480 mg d'acide [[1-(3,4-dihydroxy phényl) 2-(diphénylméthoxy) 2-oxo éthoxy] imino] [2-[(triphénylméthyl) amino] thiazol-4-yl] acétique et 120 mg d'hydrate de 1-hydroxy benzotriazole, 5 cm³ de tétrahydrofuranne et 441 cm³ de 1,3-diisopropylcarbodiimide. On évapore le solvant et chromatographie sur silice (éluant chlorure de méthylène-acétate d'éthyle (8-2)). On obtient 231 mg du produit recherché.

<u>Spectre de RMN</u> $CDCl_3$ 300 MHz ppm

| | |
|---|---|
| tBu | 1,54 (s) 1,55 (s) |
| $H_6$ et =CH-<u>$CH_2$</u>-S | 3,80 à 4,20 |
| $H_7$ cis | 5,34 (dd) 5,89 (dd) |
| O-<u>CH</u>-$C_6H_3$ (O) | 2,60 - 6,24 (dd) 8,35 (m) H mobiles |
| CH=<u>CH</u>-$CH_2$ (delta E) | 6,47 (m) |
| Les aromatiques | |
| + =<u>CH</u>-CH=$CH_2$ | 6,60 à 7,35 |
| + C(=O)-<u>OCH</u>-$(C_6H_5)_2$ | |
| $H_5$ thiazole | |

<u>STADE B</u> : (±)(cis)(Z) acide 7-[[(2-amino 4-thiazolyl) [[carboxy (3,4-dihydroxy phényl) méthoxy) imino] acétyl] amino] 8-oxo 3-[3-[(1,3,4-thiadiazol-2-yl) thio] 1-(E)-propényl 4-thia 1-aza bicyclo[4,2,0]oct-2-ène-2-carboxylate (trifluoroacétate)

On agite à 20°C pendant 2 h 30 un mélange de 215 mg du produit obtenu au stade A ci-dessus, avec 1 cm³ d'acide trifluoroacétique à 10 % d'anisole. On ajoute 10 cm³ d'éther, agite 10', essore, lave à l'éther et sèche. On obtient 120 mg du produit recherché.

Spectre de RMN DMSO 300 MHz ppm

| | |
|---|---|
| $-S-\underline{CH_2}-CH$ | 2,30 à 3,08 |
| $NH-\underline{CH}-CH$ | 5,57 |
| $NH-CH-\underline{CH}$ | 4,12 |
| $S-\underline{CH_2}-CH=CH$ | 4,12 |
| $S-CH_2-\underline{CH}=CH$ | 6,20 |
| $S-CH_2-CH=\underline{CH}$ | 6,99 (dd J=15,5 et 10) |
| 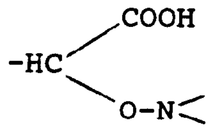 | 5,27 et 5,33 |
| Aromatiques | 6,71 à 7,50 |
| >NH | 9,28 |
| N=$\underline{CH}$ (\| S) | 9,54 |

**EXEMPLE 3 : (cis)(Z) tétrafluoroborate de 5-[[7-[[[2-amino 4-thiazolyl] [carboxy (3,4-dihydroxy phényl) méthoxy] imino] acétyl] amino] 2-carboxy 8-oxo 4-thia 1-aza bicyclo[4,2,0] oct-2-èn-3-yl] méthyl] thiéno[2,3-b]pyridinium (trifluoroacétate)**

STADE A : tétrafluoroborate de [6S-[6alpha,7béta(Z)]] 4-[[7-[[[[(3,4-dihydroxy phényl) [(diphénylméthoxy) carbonyl] méthoxy] imino] [2-[(triphénylméthyl) amino] 4-thiazolyl] acétyl] amino] 2-[(1,1-diméthyléthoxy) carbonyl] 8-oxo 4-thia 1-aza bicyclo[4,2,0]oct-2-èn-3-yl] méthyl] thiéno[2.3-b]pyridinium.

A un mélange de 57 mg de tétrafluoroborate d'argent, 53 mg de thiéno[2,3-b]pyridine et 20 cm³ de chlorure de méthylène, on ajoute 200 mg du produit obtenu au stade A de l'exemple 1. On agite 1 heure, puis ajoute à nouveau 57 mg de tétrafluoroborate d'argent et 53 mg de thiéno[2,3-b]pyridine. On agite 1 heure et évapore le solvant. On chromatographie le résidu sur silice (éluant chlorure de méthylène-méthanol 85/15) on obtient 123 mg du produit recherché.

STADE B : (cis)(Z) tétrafluoroborate de 5-[[7-[[[2-amino 4-thiazolyl] [carboxy (3,4-dihydroxy phényl) méthoxy] imino] acétyl] amino] 2-carboxy 8-oxo 4-thia 1-aza bicyclo[4,2,0] oct-2-èn-3-yl] méthyl] thiéno[2,3-b]pyridinium (trifluoroacétate)

On agite pendant 2 heures, 123 mg du produit obtenu ci-dessus avec 1 cm³ d'acide trifluoroacétique à 10 % d'anisole. On ajoute 10 cm³ d'un mélange 1-1 d'éther éthylique et isopropylique et agite pendant 30 minutes. On essore, lave et sèche. On obtient 81 mg du produit recherché.

Spectre de RMN DMSO 300 MHz ppm

| | |
|---|---|
| -S-$\underline{CH_2}$ | 2,8 à 3,4 (m) |
| -S-$CH_2$-$\underline{CH}$ | 3,89 (m) |
| NH-$\underline{CH}$< | 5,59 (dd) |
| 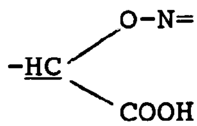 | 5,25 (s) (70 % S) 5,32 (s) (30 % R) |
| les H du phényl | 6,6 à 6,8 (m) |
| -S-$\underline{CH}$=CH- | 7,91 (d,j = 6 Hz) |
| -S-CH=$\underline{CH}$- | 8,29 (d) |
| >N=$\underline{CH}$-CH=CH | 9,24 (m) |
| >N=CH-$\underline{CH}$=CH | 8,19 (m) |
| >N=CH-CH=$\underline{CH}$ | 9,14 (m) |
| H mobiles | 7,28 (m) 9,00 (m) |

**PREPARATION DE L'EXEMPLE 4 : [6S-[3(E)6alpha,7béta(Z)]] 7-[[[[[3,4-bis[(2-méthoxyéthoxy) méthoxy] phényl] [(diphénylméthoxy) carbonyl] méthoxy] imino] [2-[(triphénylméthyl) amino] 4-thiazolyl] acétyl] amino] 3-[3-iodo 1-propényl] 8-oxo 4-thia 1-aza bicyclo[4,2,0]oct-2-ène-2-carboxylate de 1,1-diméthyléthyle**

STADE A : [6S-[3(E)6alpha,7béta(Z)]] 7-[[[[[3,4-bis[(2-méthoxyéthoxy) méthoxy] phényl] [(diphénylméthoxy) carbonyl] méthoxy] imino] [2-[(triphénylméthyl) amino] 4-thiazolyl] acétyl] amino] 3-[3-hydroxy 1-propényl] 8-oxo 4-thia 1-aza bicyclo[4,2,0]oct-2-ène-2-carboxylate de 1,1-diméthyléthyle

A une solution de 1 g d'acide [[1-(3,4-bis[(2-méthoxyéthoxy) méthoxy] phényl] 2-(diphénylméthoxy) 2-oxo éthoxy] imino] [2-[(triphénylméthyl) amino] thiazol-4-yl] acétique et 366 mg de 3-hydroxy 1-propényl 7-amino 8-oxo 4-thia 1-aza bicyclo[4,2,0]oct-2-ène-2-carboxylate de 1,1-diméthyl éthyle dans 10 $cm^3$ de chlorure de méthylène, on ajoute à -10°C, une solution de 0,324 $cm^3$ de N,N-diisopropylcarbodiimide dans 1 $cm^3$ de chlorure de méthylène, on agite à -20°C pendant 30 minutes, on chromatographie le milieu réactionnel sur silice (éluant acétate d'éthyle) et obtient 0,756 g du produit recherché.

Spectre IR ($CHCl_3$)

| | |
|---|---|
| OH | à 3610 $cm^{-1}$ |
| =C-NH | 3440-3402 $cm^{-1}$ |
| >C=O | 1777-1730-1700-1662 $cm^{-1}$ |
| Aromatique + C=C + Amide II | 1596-1565-1526-1497 $cm^{-1}$ |

STADE B : [6S-[3(E)6alpha,7béta(Z)]] 7-[[[[[3,4-bis[(2-méthoxyéthoxy) méthoxy] phényl] [(diphénylméthoxy) carbonyl] méthoxy] imino] [2-[(triphénylméthyl) amino] 4-thiazolyl] acétyl] amino] 3-[3-iodo 1-propényl] 8-oxo 4-thia 1-aza bicyclo[4,2,0]oct-2-ène-2-carboxylate de 1,1-diméthyléthyle

A un mélange de 0,250 g du produit obtenu au stade A ci-dessus, 5 $cm^3$ de chlorure de méthylène et 49 mg de iodure de tétrabutyl ammonium, on ajoute 0,047 $cm^3$ de 2,6 lutidine puis refroidit la solution à -70°C et on ajoute une solution de 1,44 $cm^3$ d'anhydride trifluorométhane sulfonique dans 0,5 $cm^3$ de chlorure de méthylène, agite 5 minutes à -70°C. On ajoute ensuite 10 $cm^3$ d'une solution aqueuse 0,2 N de thiosulfate de sodium, on agite 5 minutes puis extrait avec du chlorure de méthylène. On lave, sèche et évapore le solvant. On

chromatographie le résidu sur silice (éluant chlorure de méthylèneacétate d'éthyle (8-2)). On obtient 177 mg du produit recherché.

| | |
|---|---|
| <u>Spectre de RMN</u> $CDCl_3$ (300 MHz) ppm | |
| $CO_2tBu$ | 1,54 (s) 1,56 (s) |
| $CH_2$ en 1 | 2,30 à 2,90 (m) |
| 2 $OCH_3$ | 3,24 (s) 3,33 (s) 3,37 (s) 3,38 (s) |
| les 2 $O-CH_2-CH_2-O$ | 3,42 (m) à 3,60 (m) 4H |
| | 3,70 (m) à 3,90 (m) 4H |
| $H_6$ | 3,97 (m) |
| $CH-\underline{CH_2}-X$ | 4,06 (dd) |
| les 2-$C_6H_5-O-\underline{CH_2}-O$ | 5,25 à 5,35 (m) |
| $H_7$ | 5,61 (m) |
| $C_6H_5-\underline{CH}-O$ | 5,96 (s) 6,06 (s) |
| $=\underline{CH}-CH_2-$(delta E) | 6,35 (dt dédoublé) |
| ≃ 33 à 34 H | |
| H arome, $CO_2-\underline{CH}-(C_6H_5)_2$ | 6,58 à 7,35 (m) |
| les autres $CH=H_5$ thiazole | |
| $CO-\underline{NH}-CH$ | 7,87 (d) 8,07 (d) |

<u>EXEMPLE 4</u> : **(±)(cis)(Z) sel interne de 5-[3-[7-[[(2-amino 4-thiazolyl) [[carboxy (3,4-dihydroxy phényl) méthoxy] imino] acétyl] amino] 2-carboxy 8-oxo 4-thia 1-aza bicyclo-[4,2,0]oct-2-èn-3-yl] 2-propényl] thiazolo[4,5-c]pyridinium**

<u>STADE A</u> : iodure de [6S-[3(E)6alpha,7béta(Z)]] 5-[3-[7-[[[[[3,4-bis[(2-méthoxyéthoxy) méthoxy] phényl] [(diphénylméthoxy) carbonyl] méthoxy] imino] [2-[(triphénylméthyl) amino] 4-thiazolyl] acétyl] amino] 2-[(1,1-diméthyléthoxy) carbonyl] 8-oxo 4-thia 1-aza bicyclo[4,2,0]oct-2-èn-3-yl] 2-propényl] thiazolo[4,5- c] pyridinium

On agite 24 heures un mélange de 107 mg de [6S-[3(E)6alpha,7béta(Z)]] 7-[[[[[3,4-bis[(2-méthoxyéthoxy) méthoxy] phényl] [(diphénylméthoxy) carbonyl] méthoxy] imino] [2-[(triphénylméthyl) amino] 4-thiazolyl] acétyl] amino] 3-[3-iodo 1-propényl] 8-oxo 4-thia 1-aza bicyclo[4,2,0]oct-2-ène-2-carboxylate de 1,1-diméthyléthyle, produit obtenu au stade B de la préparation de l'exemple 4 et 11,4 mg de thiazolo[4,5- c] pyridine avec 0,4 $cm^3$ de diméthyl sulfoxyde. On évapore à sec et chromatographie le résidu sur silice (éluant : chlorure de méthylène-méthanol (92-8)) on obtient 82 mg de produit utilisé tel quel pour le stade suivant.

<u>STADE B</u> : (±)(cis)(Z) sel interne de 5-[3-[7-[[(2-amino 4-thiazolyl) [[carboxy (3,4-dihydroxy phényl) méthoxy] imino] acétyl] amino] 2-carboxy 8-oxo 4-thia 1-aza bicyclo[4,2,0]oct-2-èn-3-yl] 2-propényl] thiazolo[4,5- c] pyridinium

A une solution de 107 mg de produit obtenu comme au stade A, 2,2 $cm^3$ de chlorure de méthylène et 0,45 $cm^3$ d'anisole, on ajoute à 0°C, une solution refroidie à 0°C de 4,3 $cm^3$ d'acide trifluoroacétique dans 2,2 $cm^3$ de chlorure de méthylène. On agite 1 heure à 0°C, concentre sous pression réduite, reprend le résidu à l'éther et essore. On obtient 45 mg de produit que l'on agite pendant 1 heure à 0°C avec 1,6 $cm^3$ d'une solution d'acide trifluoroacétique à 10 % d'anisole, on traite comme ci-dessus et obtient 37,2 mg du produit attendu.

Spectre de RMN DMSO 400 MHz

| | |
|---|---|
| S-$\underline{CH_2}$-CH | 2,71 - 2,89 - 3,06 - 3,21 (m) |
| $H_6$ | 3,91 - 3,98 (m) |
| N-O-$\underline{CH}$-$C_6H_5$ | 5,26 - 5,32 (s) |
| $^{\ominus}$N-$CH_2$-C | 5,53 (m) |
| $H_7$ | 5,57 (m) |
| CH= | 6,38 (m) delta E |
| Aromatique + | |
| Thiazole + | 6,7 à 7,35 (m) |
| Autres éthyléniques | |
| $H_6$' et $H_7$' | ≃ 8,93 ≃ 8,85 (dd) |
| $H_4$' et $H_2$' | 9,93 - 10,05 (s,d) |
| Proton mobile | 7,30 - 9,10 ; 9,28 et 12,95 |

**EXEMPLE 5 : (±)(cis)(Z) sel interne de 4-[3-[7-[[(2-amino 4-thiazolyl) [[carboxy (3,4-dihydroxy phényl) méthoxy] imino] acétyl] amino] 2-carboxy 8-oxo 4-thia 1-aza bicyclo-[4,2,0]oct-2-èn-3-yl] 2-propényl] thiéno[3,2-b]pyridinium**

STADE A : trifluorométhanesulfonate de [6S[3(E)6alpha,7béta(Z)]] 4-[3-[7-[[[[[3,4-bis[(2-méthoxyéthoxy) méthoxy] phényl] [(diphénylméthoxy) carbonyl] méthoxy] imino] [2-[(triphénylméthyl) amino] 4-thiazolyl] acétyl] amino] 2-[(1,1-diméthyléthoxy) carbonyl] 8-oxo 4-thia 1-aza bicyclo-[4,2,0]oct-2-èn-3-yl] 2-propényl] thiéno[3,2-b]pyridinium

A une solution de 240 mg de produit obtenu au stade A de la préparation de l'exemple 4 et 104,8 mg de thiéno[3,2-b]pyridine, avec 8 $cm^3$ de chlorure de méthylène, on ajoute à - 60°C, 0,079 $cm^3$ d'anhydridre trifluorométhane sulfonique, on agite 5 minutes à -60°C, puis laisse revenir à 0°C en 30 minutes. On ajoute 1 $cm^3$ d'eau, décante et après séchage évapore à sec sous pression réduite.

On chromatographie le résidu (423 mg) sur silice (éluant chlorure de méthylène-méthanol (95-5)). On obtient 210 mg de produit recherché que l'on utilise tel que pour le stade suivant.

STADE B : (±)(cis)(Z) sel interne de 4-[3-[7-[[(2-amino 4-thiazolyl) [[carboxy (3,4-dihydroxy phényl) méthoxy] imino] acétyl] amino] 2-carboxy 8-oxo 4-thia 1-aza bicyclo[4,2,0]oct-2-èn-3-yl] 2-propényl] thiéno[3,2-b]pyridinium

A une solution de 210 mg du produit obtenu au stade A, 5 $cm^3$ de chlorure de méthylène et 1 $cm^3$ d'anisole, on ajoute à 0°C une solution de 10 $cm^3$ d'acide trifluoroacétique dans 5 $cm^3$ de chlorure de méthylène refroidie à 0°C. On agite pendant 1 heure à 0°C. On évapore les solvants et reprend le résidu avec 10 $cm^3$ d'éther. On essore, lave et sèche. On obtient 68 mg du produit attendu.

<u>Spectre de RMN</u> DMSO 250 MHz

| | |
|---|---|
| $H_6$ | 3,96 (m) |
| $C_6H_5$-<u>CH</u>-O | 5,25 - 5,32 |
| $H_7$ | 5,56 (d dédoublé après échange) |
| $^{\oplus}$N-$CH_2$ | 5,74 (raie large) |
| 1H éthylénique DELTA E | 6,33 (d dédoublé) |
| $H_6$' et $H_3$' ou $H_2$' | 8,05 à 8,15 (m) (2H) |
| $H_3$' ou $H_2$' | 8,86 (d) |
| $H_5$' ou $H_7$' | 9,18 (d,l) et 9,37 (d) |
| Protons mobiles | 7,31 |

| | |
|---|---|
| Aromatiques, $H_5$ du thiazole et l'autre éthylénique | 6,7 à 7,4 (m) |
| S-$CH_2$ (en partie masqué par le diméthyle sulfoxyde) | $\simeq$ 2,4 à 2,5 |

**<u>EXEMPLE 6</u> : (±)(cis)(Z) sel interne de 7-[3-[7-[[(2-amino 4-thiazolyl) [[carboxy (3,4-dihydroxy phényl) méthoxy] imino] acétyl] amino] 2-carboxy 8-oxo 4-thia 1-aza bicyclo[4,2,0]oct-2-èn-3-yl] 2-propényl] thiéno[3,2-b]pyridinium**

<u>STADE A</u> : trifluorométhanesulfonate de [6S[3(E)6alpha,7béta(Z)]] 7-[3-[7-[[[[[3,4-bis[(2-méthoxyéthoxy) méthoxy] phényl] [(diphénylméthoxy) carbonyl] méthoxy] imino] [2-[(triphénylméthyl) amino] 4-thiazolyl] acétyl] amino] 2-[(1,1-diméthyléthoxy) carbonyl] 8-oxo 4-thia 1-aza bicyclo[4,2,0]oct-2-èn-3-yl] 2-propényl] thiéno[3,2-b]pyridinium

A un mélange de 86 mg de thiéno[2,3-b]pyridine, 6 $cm^3$ de chlorure de méthylène et 200 mg du produit obtenu au stade A de la préparation de l'exemple 4, on ajoute à -70°C, 0,3 $cm^3$ d'une solution constituée de 1 $cm^3$ d'anhydride trifluorométhane sulfonique dans 4 $cm^3$ de chlorure de méthylène. On agite 10 minutes à -70°C puis laisse la température revenir à 20°C en 1 heure. On ajoute 2 $cm^3$ d'eau, décante, sèche et concentre sous pression réduite. On chromatographie le résidu sur silice (éluant chlorure de méthylène-méthanol (92-8)). On obtient 123 mg du produit recherché que l'on utilise tel quel pour le stade suivant.

<u>STADE B</u> : (±)(cis)(Z) sel interne de 7-[3-[7-[[(2-amino 4-thiazolyl) [[carboxy (3,4-dihydroxy phényl) méthoxy] imino] acétyl] amino] 2-carboxy 8-oxo 4-thia 1-aza bicyclo[4,2,0]oct-2-èn-3-yl] 2-propényl] thiéno[3,2-b]pyridinium

A une solution de 139 mg du produit obtenu au stade A dans 3,5 $cm^3$ de chlorure de méthylène et 0,69 $cm^3$ d'anisole, on ajoute à 0°C, 6,95 $cm^3$ d'acide trifluoroacétique en solution dans 3,5 $cm^3$ de chlorure de méthylène. On agite 1 h 15 mn à 0°C, on concentre à sec sous pression réduite, on reprend l'extrait sec avec de l'éther, essore, lave et sèche et obtient 70 mg de produit que l'on reprend à nouveau dans 1,75 $cm^3$ de chlorure de méthylène et 0,34 $cm^3$ d'anisole. On ajoute à 20°C, 3,48 $cm^3$ d'acide trifluoroacétique dans 1,75 $cm^3$ de chlorure de méthylène, on agite 10 minutes à 20°C puis évapore à sec sous pression réduite, reprend l'extrait sec à l'éther, puis essore, lave et sèche. On obtient 43 mg de produit recherché.

Spectre de RMN DMSO 300 MHz ppm

| | |
|---|---|
| $CH_2S$ | 2,70 à 3,10 |
| $H_6$ | 3,93 |
| $OCH_2O$ des MEM | 5,0 à 5,10 |
| $C_6H_5$-CHO<br>\|<br>CO | 5,25 (s) 5,32 (s) |
| $H_7$ (cis/$H_6$) | 5,58 (d après échange) |
| =C-$CH_2N^{\oplus}$<br>\| | 5,59 (d après échange) |
| | 5,70 |
| $H_5$ thiazole | 6,69 ou 6,80 (s) |
| CH=CH + | |
| Aromatique + | 6,68 à 7,31 |
| $NH_2$ | |
| CH=<u>CH</u>-$CH_2$ | 6,19 (dt) |
| $H_2$' et $H_3$' | 7,89 (d) et 8,28 (d) |
| $H_5$' | 8,15 (t) |
| $H_4$' | 9,09 (d) |
| $H_6$' | 9,20 (d) |
| Les C-NH-CH<br>‖<br>O | 9,29 (m) |

**<u>EXEMPLE 7</u> : (±)(cis)(Z) sel interne de 7-[3-[7-[[(2-amino 4-thiazolyl) [[carboxy (3,4-dihydroxy phényl) méthoxy] imino] acétyl] amino] 2-carboxy 8-oxo 4-thia 1-aza bicyclo[4,2,0]oct-2-èn-3-yl] 2-propényl] thiéno[2,3-b]pyridinium**

A une solution de 123 mg de produit obtenu au stade A de l'exemple 6, dans 2,8 $cm^3$ de chlorure de méthylène et 0,6 $cm^3$ d'anisole on ajoute à 0°C, 6,15 $cm^3$ d'acide trifluoroacétique. On agite 15' à 0°C, puis 5 heures à 20°C. On concentre à sec sous pression réduite, reprend à l'éther, essore, lave et sèche. On obtient 57 mg de produit que l'on dissout dans un mélange 50-50 d'acétonitrile et d'eau avec 50 microlitres de triéthylamine. Cette solution est chromatographiée sur support Bondapack $C_{18}$ (éluant : acétonitrile-eau (50-50)) on évapore à faible volume sous pression réduite puis lyophilise. On obtient 45 mg de produit recherché auquel on ajoute 15 mg provenant d'une première opération et effectue une nouvelle chromatographie sur support RP18 en éluant acétonitrile-eau (50-50). On concentre à volume réduit sous pression réduite, ajoute un peu d'eau et lyophilise, on obtient 33 mg de produit recherché.

<u>Spectre de RMN</u> DMSO 300 MHz ppm

| | |
|---|---|
| $CH_2S$ | 3,75 (m) |
| $H_6$ | 3,96 (m) |
| $C_6H_5$-CH-CO | 5,23 (s) - 5,36 (s) |
| $H_7$ | 5,42 (dd, d après échange) |
| =CH-<u>CH</u>$_2$-$N^{\oplus}$ | 5,57 (m) |
| =<u>CH</u>-$CH_2$ | 5,90 (m) |
| H aromatique + $H_5$ thiazole + H éthylénique | 6,60 à 7,40 (m) |
| $H_5$' | 8,12 (m) |
| $H_2$' et $H_3$' | 7,87 (d) 8,26 (d) |
| $H_4$' | 9,05 |
| $H_6$' | 9,20 |

**<u>EXEMPLE 8</u> : (±)(cis)(Z) sel interne de 2-amino 5-[3-[7-[[(2-amino 4-thiazolyl) [[carboxy (3,4-dihydroxy phényl) méthoxy] imino] acétyl] amino] 2-carboxy 8-oxo 4-thia 1-aza bicyclo-[4,2,0]oct-2-èn-3-yl] 2-propényl] thiazolo[4,5-c]pyridinium**

<u>STADE A</u> : iodure de [6S[3(E)6alpha,7béta(Z)]] 2-amino 5-[3-[7-[[[[[3,4-bis[(2-méthoxyéthoxy) méthoxy] phényl] [(diphénylméthoxy) carbonyl] méthoxy] imino] [2-[(triphénylméthyl) amino] 4-thiazolyl] acétyl] amino] 2-[(1,1-diméthyléthoxy) carbonyl] 8-oxo 4-thia 1-aza bicyclo[4,2,0]oct-2-èn-3-yl] 2-propényl] thiazolo[4,5-c] pyridinium

On agite 7 heures un mélange constitué par 200 mg du produit obtenu au stade B de la préparation de l'exemple 4 avec 36,2 mg d'aminothiazolopyridine et 3 $cm^3$ de diméthyl sulfoxyde. Après évaporation du solvant sous pression réduite on chromatographie le résidu sur silice (éluant : chlorure de méthylène-méthanol (9-1)), on obtient 156 mg de produit recherché.

<u>STADE B</u> : (±)(cis)(Z) sel interne de 2-amino 5-[3-[7-[[(2-amino 4-thiazolyl) [[carboxy (3,4-dihydroxy phényl) méthoxy] imino] acétyl] amino] 2-carboxy 8-oxo 4-thia 1-aza bicyclo-[4,2,0]oct-2-èn-3-yl] 2-propényl] thiazolo[4,5-c] pyridinium

A une solution de 155,6 mg du produit obtenu au stade A dans 4 $cm^3$ de chlorure de méthylène et 0,75 $cm^3$ d'anisole, on ajoute à 0°C une solution refroidie à 0°C de 4 $cm^3$ de chlorure de méthylène et 7,4 $cm^3$ d'acide trifluoroacétique. On agite 1 heure à 0°C, évapore à sec sous pression réduite et cristallise le résidu dans l'éther. On obtient 57 mg de produit que l'on traite à 0°C pendant 1 heure avec 2 $cm^3$ d'une solution d'acide trifluoroacétique à 10 % d'anisole, on traite comme ci-dessus et répète encore une fois cette opération, on obtient ainsi 31,5 mg de produit recherché.

Spectre de RMN DMSO 400 MHz ppm

| | |
|---|---|
| $H_7$ dédoublé | 5,54 (dd) 5,59 (dd) |
| 1-CH delta E | 6,33 (m) |
| $C_6H_5$-CH-O | 5,26 (s) - 5,33 (s) |
| $N^{\oplus}$-$CH_2$ | 5,30 (m) |
| Aromatique + $H_5$ thiazole + H éthylénique | 6,7 à 7,3 (m) |
| S-$\underline{CH_2}$-CH | 2,3 (m) 2,69 (m) 2,89 (m) 3,06 (m) |
| $H_6$' et $H_7$' | 8,43 (m) 8,49 (m) |
| $H_4$' | 9,02 (d) |
| NH-CH | 9,26 et 9,30 (d) |
| Protons mobiles | 12,9 et 13,5 |

**EXEMPLE 9 : On a réalisé des préparations pour injections de formule :**

- Produit de l'exemple 3 500 mg
- Excipient aqueux stérile q.s.p. 5 $cm^3$

**ETUDE PHARMACOLOGIQUE DES PRODUITS DE L'INVENTION**

Activité in vitro, méthode des dilutions en milieu liquide.

On prépare une série de tubes dans lesquels on répartit une même quantité de milieu nutritif stérile. On distribue dans chaque tube des quantités croissantes du produit à étudier, puis chaque tube est ensemencé avec une souche bactérienne.

Après incubation de vingt-quatre heures à l'étuve à 37°C, l'inhibition de la croissance est appréciée par transillumination, ce qui permet de déterminer les concentrations minimales inhibitrices (C.M.I. exprimées en µg/$cm^3$).

Les résultats suivants ont été obtenus :

| SOUCHES | Produits de l'ex. 1 | Produit de l'ex. 3 |
|---|---|---|
| Pseudomonas aeruginosa 1771 | 1,25 | 0,6 |
| Pseudomonas aeruginosa 1771 m | 0,6 | 0,15 |
| Pseudomonas aeruginosa ATCC 9027 | 1,25 | 0,6 |

**Revendications**

**1)** Les produits de formule générale (I) :

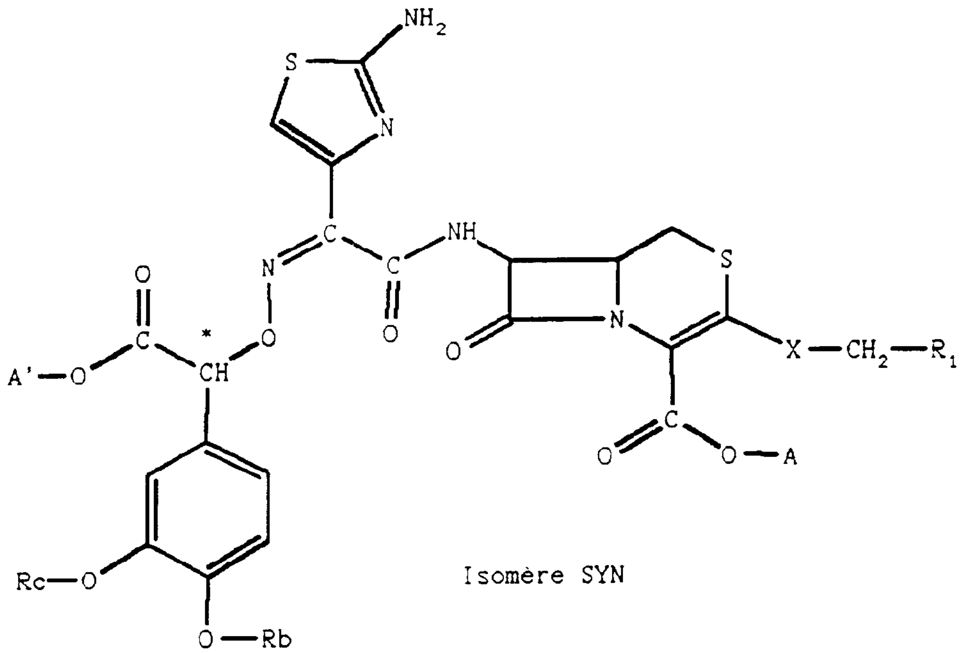

(I)

Isomère SYN

isomère syn, sous forme R ou S ou d'un mélange R, S, formule dans laquelle :
$R_1$ représente soit un radical choisi parmi les radicaux suivants :

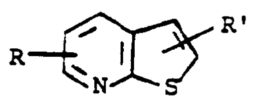
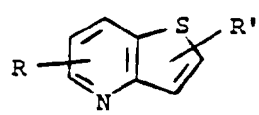
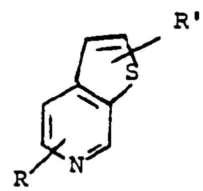
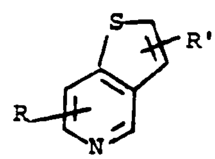

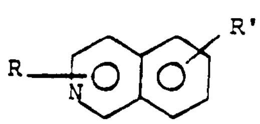
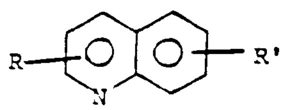
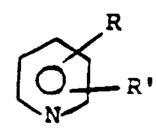
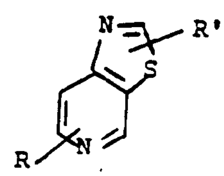

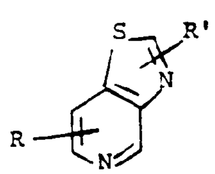
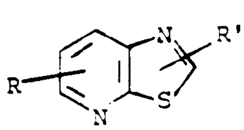
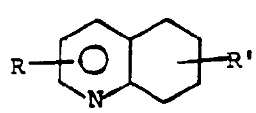
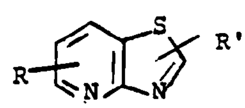

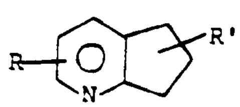
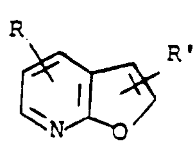
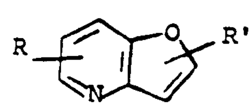
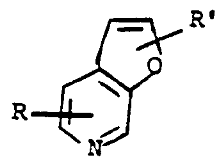

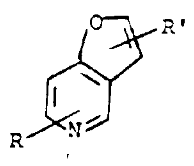
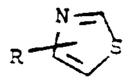
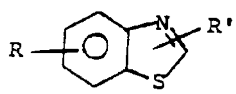
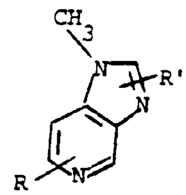
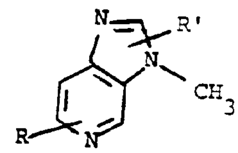

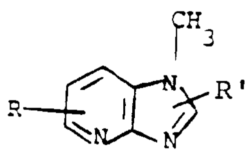
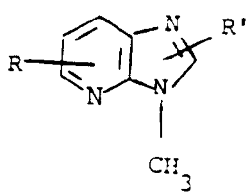
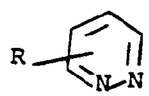
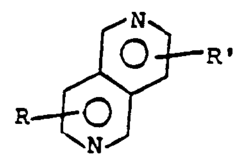

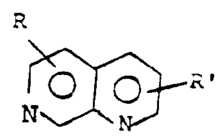
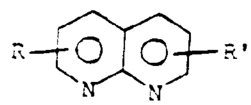
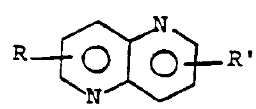
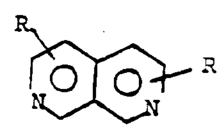

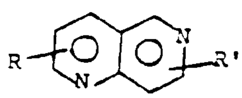
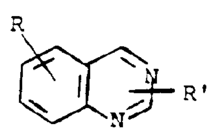
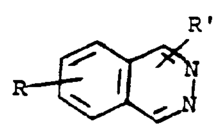
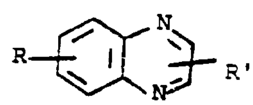

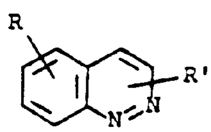
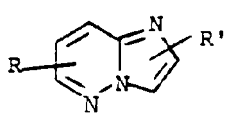
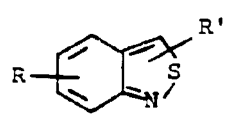
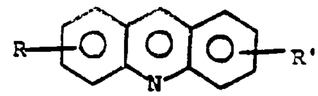

ou

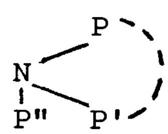
,

sous forme d'ammonium quaternaire, ou

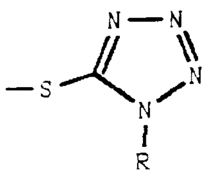 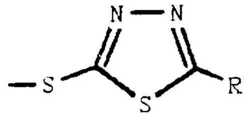

dans lesquels R et R′ identiques ou différents représentent un atome d'hydrogène, un radical alkyle renfermant de 1 à 4 atomes de carbone, un radical alcoxy renfermant de 1 à 4 atomes de carbone, un atome d'halogène, un radical $CO_2$-Q,

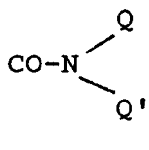 , 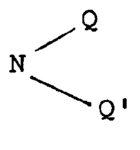 , 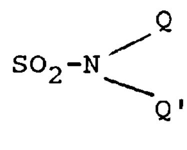 , 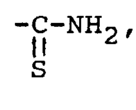

NH-CO-Q, CN, $CH_2$-CN, $CH_2$-SQ dans lesquels Q et Q′ identiques ou différents représentent un atome d'hydrogène ou un radical alkyle renfermant de 1 à 4 atomes de carbone, P, P′ et P″ identiques ou différents représentent un radical alkyle renfermant au plus 4 atomes de carbone, éventuellement substitué par un des substituants indiqués ci-dessus pour R et R′, le trait pointillé indiquant que P et P′ peuvent éventuellement former avec l'atome d'azote auquel ils sont liés, un hétérocycle à 5 ou 6 chaînons,

soit $R_1$ représente un radical -S-Het dans lequel Het représente un hétérocycle choisi parmi les radicaux thiazolyle, isothiazolyle, oxazolyle, isoxazolyle, triazolyle, thiadiazolyle, oxadiazolyle, tétrazolyle, imidazolyle, pyrazolyle, pyridinyle, pyrimidinyle, pyrazinyle, pyridazinyle, triazinyle, dihydrotriazinyle, ces radicaux étant éventuellement substitués par un ou plusieurs substituants choisis parmi les radicaux alkyle ayant de 1 à 4 atomes de carbone, de préférence méthyle, amino, alkyl ou dialkylamino hydroxy, oxo, alkylthio, carboxy ou carboxyalkyle libres, estérifiés ou salifiés.

X représente une simple liaison ou un radical

$>C = C<$

sous forme E ou Z.

$R_b$ et $R_c$, identiques ou différents représentent un atome d'hydrogène ou un groupement acyle,

A et A′ identiques ou différents représentent un atome d'hydrogène, un équivalent de métal alcalin, alcalino-terreux, de magnésium, d'ammonium ou d'une base organique aminée ou A et A′ représentent le reste d'un groupement ester facilement clivable ou $CO_2A$ représente $CO_2^{\ominus}$ ainsi que les sels des produits de formule (I) avec les acides minéraux ou organiques.

**2)** Les produits de formule générale (I) telle que définie à la revendication 1, dans laquelle $R_1$ est choisi soit parmi les radicaux :

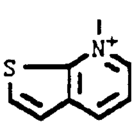 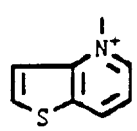 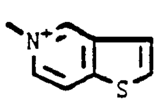 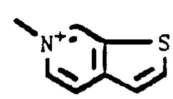 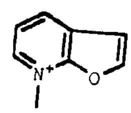 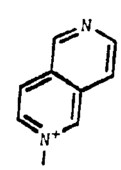

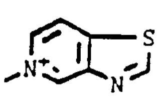 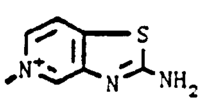 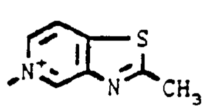 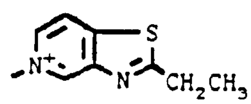 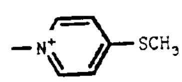

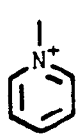 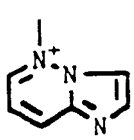 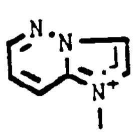 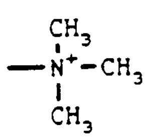 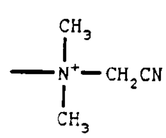

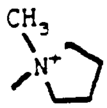 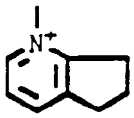 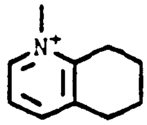 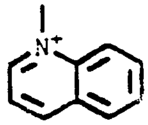 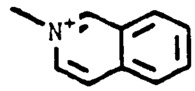 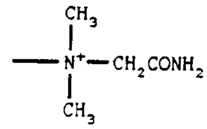

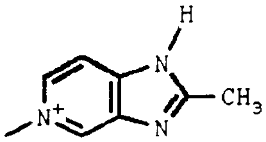 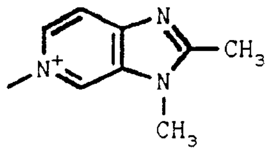 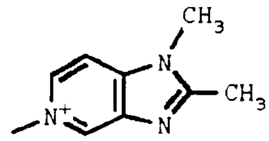 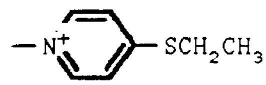

soit parmi les radicaux S-Het, dans lesquels Het représente les radicaux 1,2,4, 1,3,4 ou 1,2,3-thiadiazolyle ; thiazole-2-yle ; imidazol-2-yle ; 2-méthyl 1,3,4-thiadiazol-5-yle ; 1-méthyl tétrazol-5-yle et les radicaux :

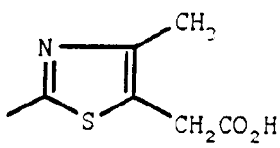 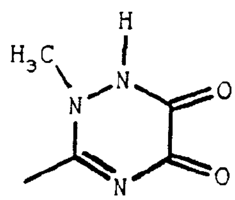 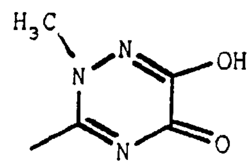

**3)** Les produits de formule générale (I) telle que définie à l'une des revendications 1 ou 2 dans laquelle $R_1$ est choisi
soit parmi les radicaux suivants :

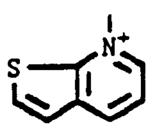 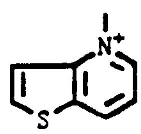 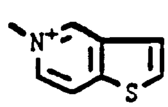 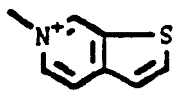

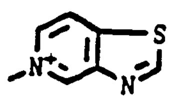 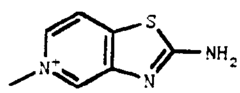 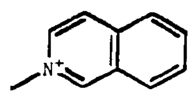

de préférence le radical :

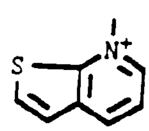

soit $R_1$ représente un radical 1,3,4-thiadiazolylthio ou méthyltétrazolylthio.

**4)** Le (S)(cis)(Z) 5-[[7-[[(2-amino 4-thiazolyl) [[carboxy-(3,4-dihydroxy phényl) méthoxy] imino] acétyl] amino] 2-carboxy 8-oxo 4-thia 1-aza bicyclo[4,2,0]oct-2-èn-3-yl] méthyl] thiéno[2,3-b]pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables.

**5)** Procédé de préparation des produits de formule (I) telle que définie à la revendication 1, caractérisé en ce que
soit l'on fait agir un produit de formule (II) :

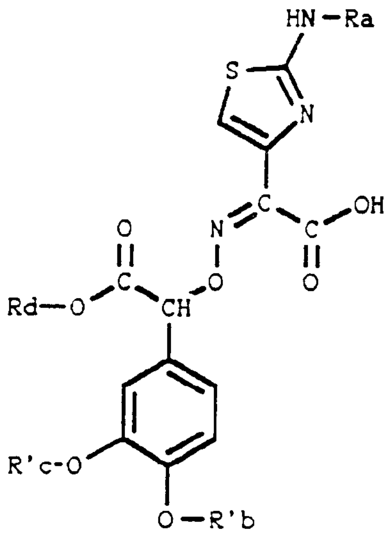

(II)

isomère syn, racémique ou optiquement actif ou un dérivé fonctionnel du produit de formule (II), dans laquelle $R_a$ représente un atome d'hydrogène ou un groupement protecteur du radical amino, $R'_b$ et $R'_c$ identiques ou différents représentent un atome d'hydrogène ou un groupement protecteur du radical hydroxyle, $R_d$ représente un atome d'hydrogène ou le reste d'un groupement ester facilement éliminable, avec un produit de formule (III) :

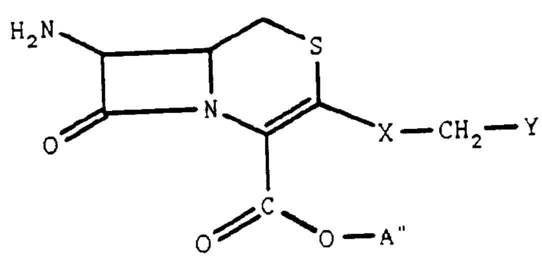

(III)

dans laquelle Y représente un radical hydroxy ou un atome d'halogène, A″ représente un atome d'hydrogène ou le reste d'un groupement ester facilement éliminable pour obtenir un produit de formule (IV) :

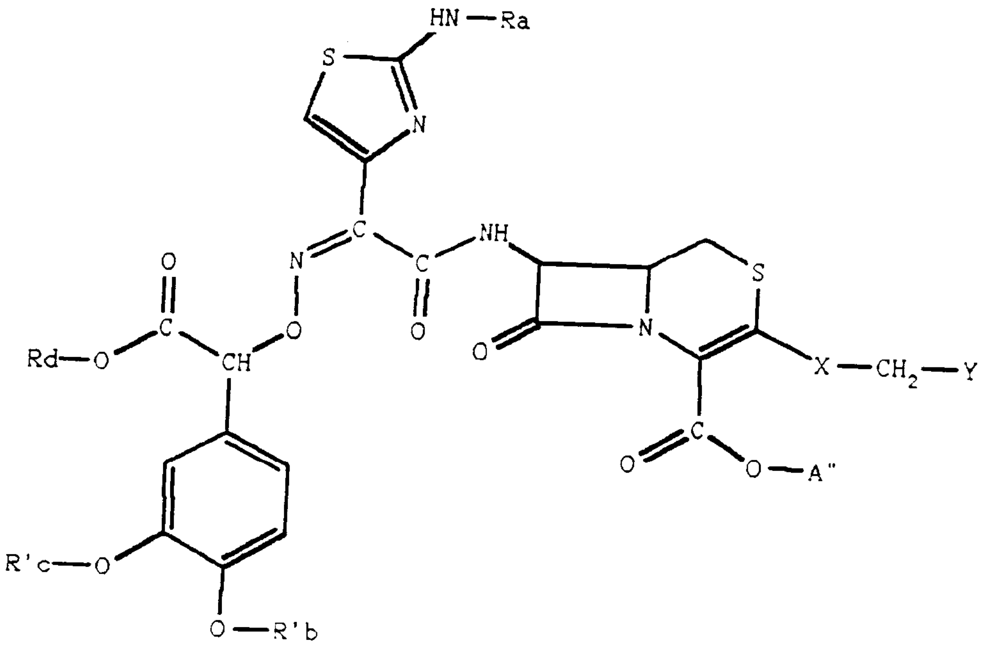

(IV)

que l'on fait réagir avec un réactif capable d'introduire le radical $R_1$ pour obtenir un produit de formule (V) :

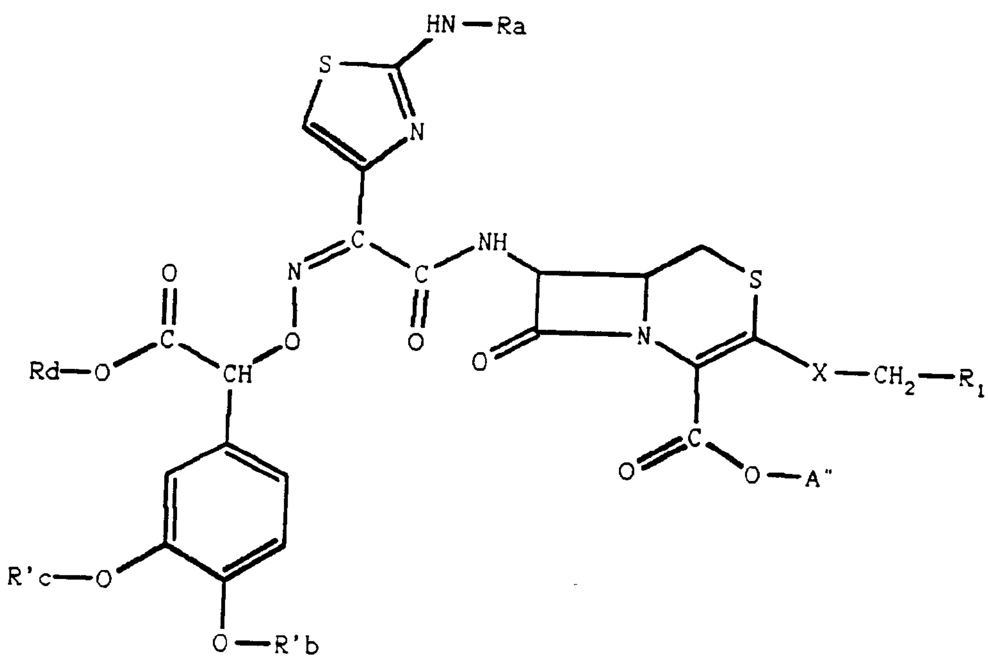

(V)

soit l'on fait réagir un produit de formule (VI) :

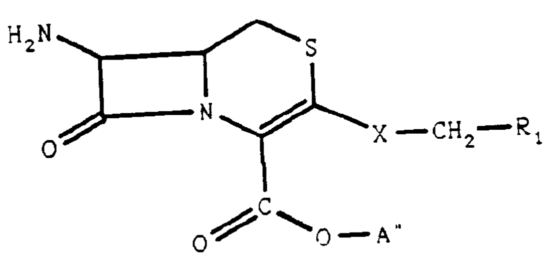

(VI)

dans laquelle X, $R_1$ et A″ ont la signification précédente avec un produit de formule (II) telle que définie ci-dessus ou un dérivé fonctionnel du produit de formule (II) pour obtenir un produit de formule (V) telle que définie ci-dessus,

produit de formule (V) que, si désiré, lorsque X représente un radical >C = C< l'on sépare en ses isomères E ou Z ou transforme les isomères Z en isomères E et produits de formule (V) que, si nécessaire ou si désiré, l'on soumet à une ou plusieurs des réactions suivantes, dans un ordre quelconque :

a) coupure par hydrolyse ou par action de la thiourée de tout ou partie des groupements esters ou des groupements de protection du radical amino ou des radicaux hydroxyles,

b) estérification ou salification par une base du ou des radicaux carboxyliques,

c) salification par un acide du radical amino,

d) séparation des produits sous forme de mélange R,S en R ou S.

**6)** A titre de médicaments, les produits répondant à la formule (I) telle que définie à la revendication 1, ainsi que leurs sels d'acides, pharmaceutiquement acceptables.

**7)** A titre de médicaments, les produits tels que définis à l'une quelconque des revendications 2 à 4 ainsi que leurs sels d'acides, pharmaceutiquement acceptables.

**8)** Compositions pharmaceutiques contenant, à titre de principe actif, au moins un médicament selon l'une des revendications 6 ou 7.

**9)** A titre de produits industriels nouveaux, les produits de formule (IV) et les produits de formule (V) dans laquelle $R_a$ représente un groupement protecteur du radical amino, les formules (IV) et (V) étant telles que définies à la revendication 5.

**Revendications pour l'Etat contractant suivant : ES**

**1.-** Procédé pour préparer des produits de formule générale (I) :

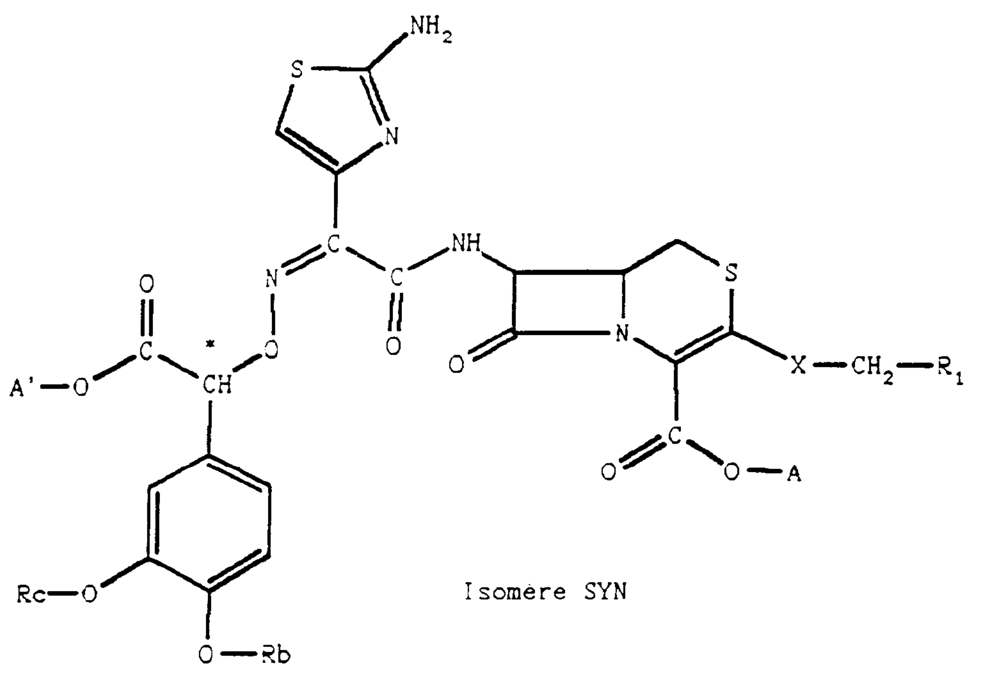

(I)

Isomère SYN

isomère syn, sous forme R ou S ou d'un mélange R, S, formule dans laquelle :

$R_1$ représente soit un radical choisi parmi les radicaux suivants :

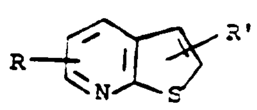 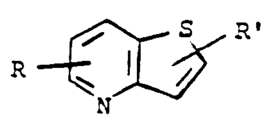 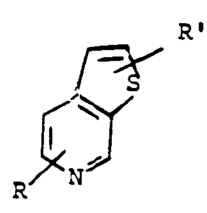 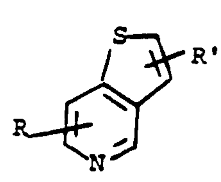

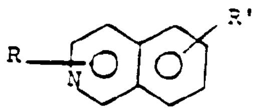
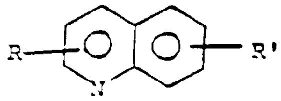
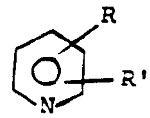
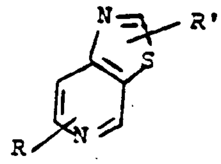

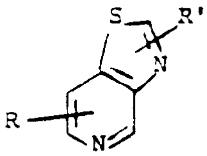
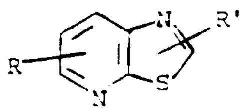
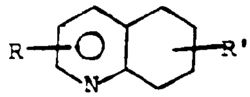
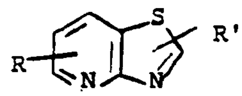

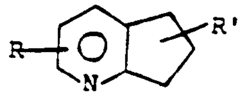
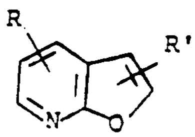
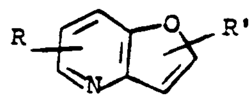
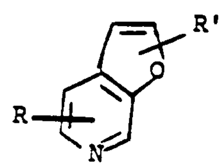

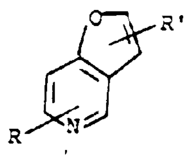
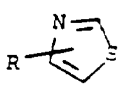
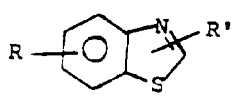
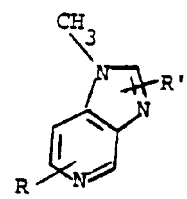
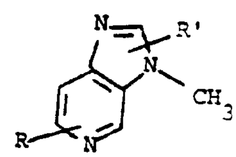

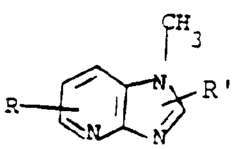
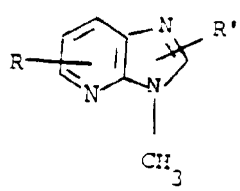
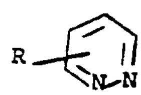
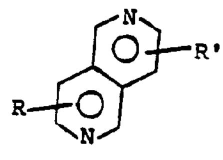

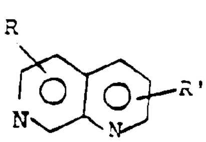
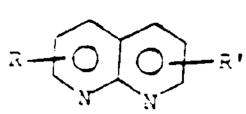
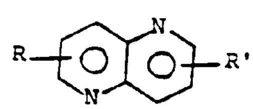
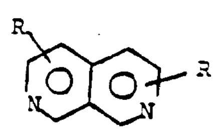

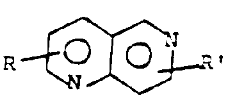
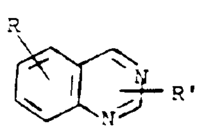
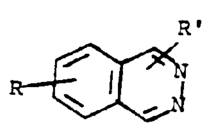
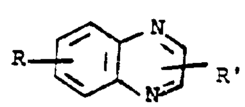

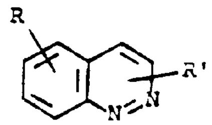 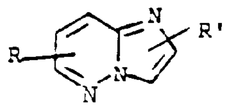 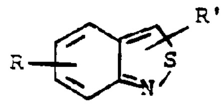 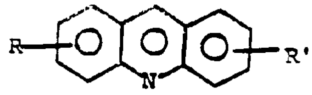

ou

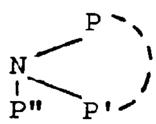 ,

sous forme d'ammonium quaternaire, ou

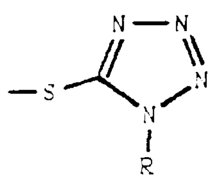 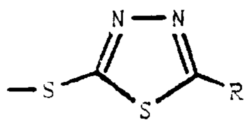

dans lesquels R et R′ identiques ou différents représentent un atome d'hydrogène, un radical alkyle renfermant de 1 à 4 atomes de carbone, un radical alcoxy renfermant de 1 à 4 atomes de carbone, un atome d'halogène, un radical $CO_2$-Q,

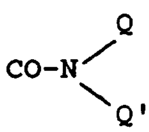 , 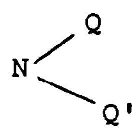 , 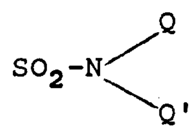 , 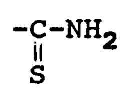 ,

NH-CO-Q, CN, $CH_2$-CN, $CH_2$-SQ dans lesquels Q et Q′ identiques ou différents représentent un atome d'hydrogène ou un radical alkyle renfermant de 1 à 4 atomes de carbone, P, P′ et P″ identiques ou différents représentent un radical alkyle renfermant au plus 4 atomes de carbone, éventuellement substitué par un des substituants indiqués ci-dessus pour R et R′, le trait pointillé indiquant que P et P′ peuvent éventuellement former avec l'atome d'azote auquel ils sont liés, un hétérocycle à 5 ou 6 chaînons,

soit $R_1$ représente un radical -S-Het dans lequel Het représente un hétérocycle choisi parmi les radicaux thiazolyle, isothiazolyle, oxazolyle, isoxazolyle, triazolyle, thiadiazolyle, oxadiazolyle, tétrazolyle, imidazolyle, pyrazolyle, pyridinyle, pyrimidinyle, pyrazinyle, pyridazinyle, triazinyle, dihydrotriazinyle, ces radicaux étant éventuellement substitués par un ou plusieurs substituants choisis parmi les radicaux alkyle ayant de 1 à 4 atomes de carbone, de préférence méthyle, amino, alkyl ou dialkylamino hydroxy, oxo, alkylthio, carboxy ou carboxyalkyle libres, estérifiés ou salifiés.

X représente une simple liaison ou un radical >C = C< sous forme E ou Z.

$R_b$et $R_c$, identiques ou différents représentent un atome d'hydrogène ou un groupement acyle,

A et A′ identiques ou différents représentent un atome d'hydrogène, un équivalent de métal alcalin, alcalino-terreux, de magnésium, d'ammonium ou d'une base organique aminée ou A et A′ représentent le reste d'un groupement ester facilement clivable ou $CO_2$A représente $CO_2^{\ominus}$ ainsi que les sels des produits de formule (I) avec les acides minéraux ou organiques, caractérisé en ce que

soit l'on fait agir un produit de formule (II) :

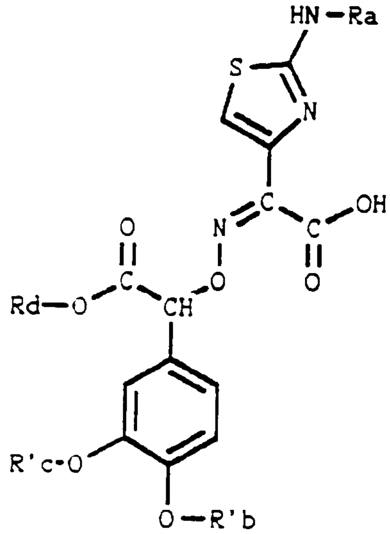

(II)

isomère syn, racémique ou optiquement actif ou un dérivé fonctionnel du produit de formule (II), dans laquelle $R_a$ représente un atome d'hydrogène ou un groupement protecteur du radical amino, $R'_b$ et $R'_c$ identiques ou différents représentent un atome d'hydrogène ou un groupement protecteur du radical hydroxyle, $R_d$ représente un atome d'hydrogène ou le reste d'un groupement ester facilement éliminable, avec un produit de formule (III) :

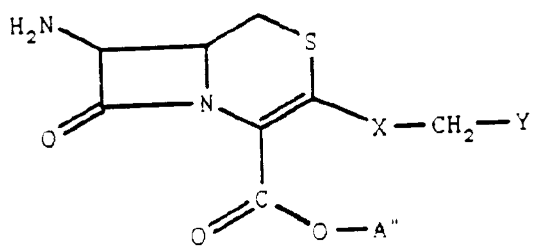

(III)

dans laquelle Y représente un radical hydroxy ou un atome d'halogène, A" représente un atome d'hydrogène ou le reste d'un groupement ester facilement éliminable pour obtenir un produit de formule (IV) :

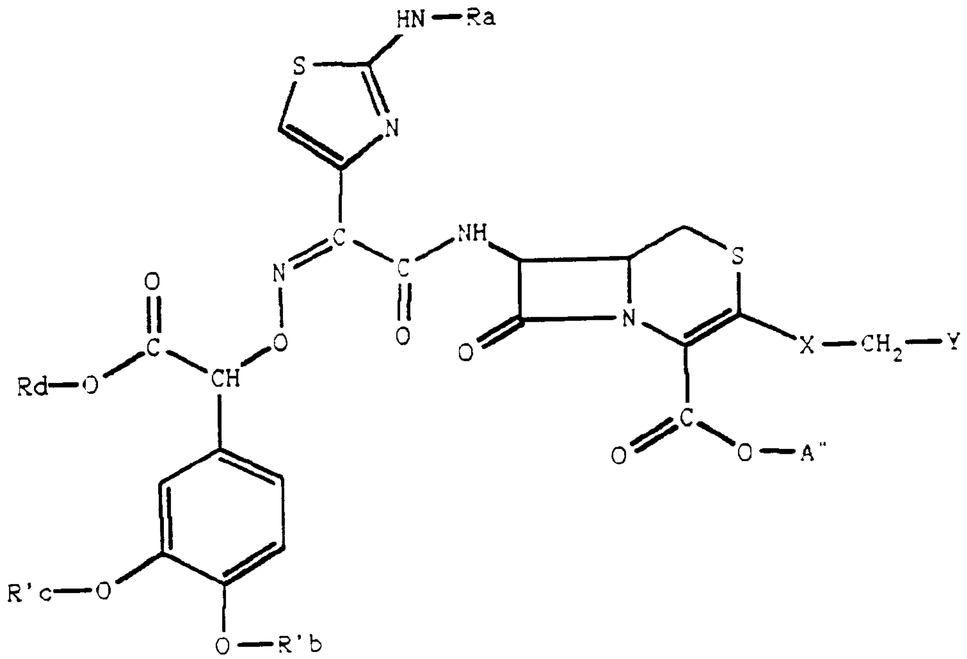

(IV)

que l'on fait réagir avec un réactif capable d'introduire le radical $R_1$ pour obtenir un produit de formule (V) :

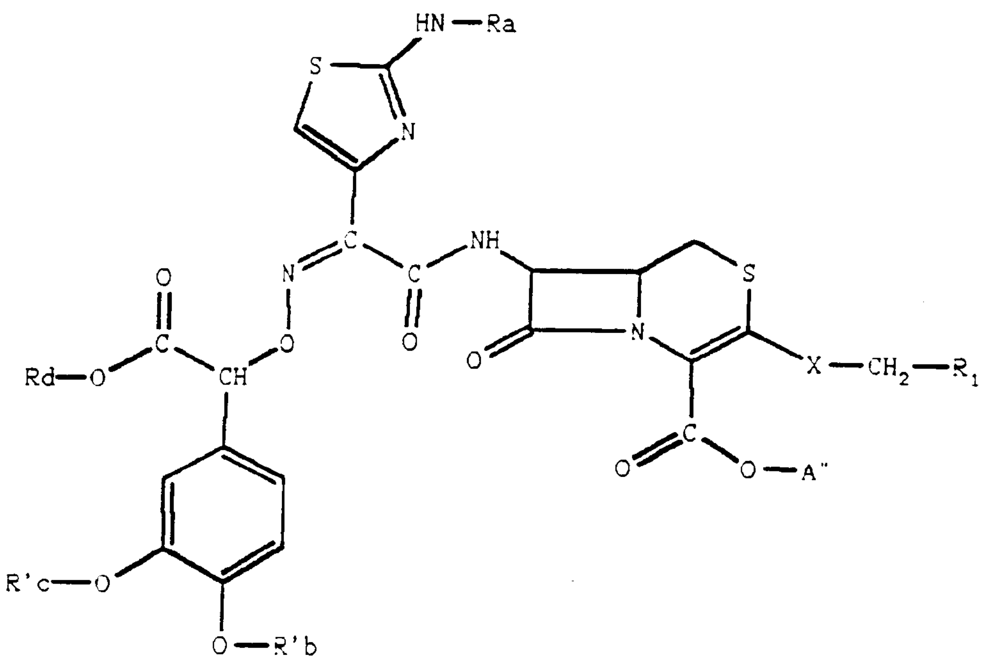

(V)

soit l'on fait réagir un produit de formule (VI) :

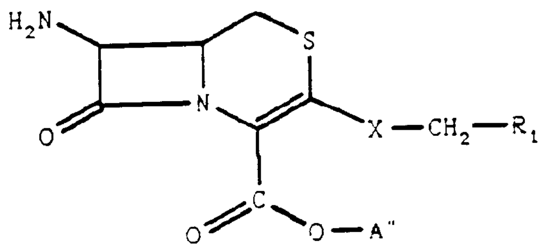

(VI)

dans laquelle X, $R_1$ et A″ ont la signification précédente avec un produit de formule (II) telle que définie ci-dessus ou un dérivé fonctionnel du produit de formule (II) pour obtenir un produit de formule (V) telle que définie ci-dessus,

produit de formule (V) que, si désiré, lorsque X représente un radical C = C l'on sépare en ses isomères E ou Z ou transforme les isomères Z en isomères E et produits de formule (V) que, si nécessaire ou si désiré, l'on soumet à une ou plusieurs des réactions suivantes, dans un ordre quelconque :

a) coupure par hydrolyse ou par action de la thiourée de tout ou partie des groupements esters ou des groupements de protection du radical amino ou des radicaux hydroxyles,

b) estérification ou salification par une base du ou des radicaux carboxyliques,

c) salification par un acide du radical amino,

d) séparation des produits sous forme de mélange R,S en R ou S.

**2.-** Procédé selon la revendication 1 pour préparer des produits de formule générale (I) telle que définie à la revendication 1, dans laquelle $R_1$ est choisi

soit parmi les radicaux :

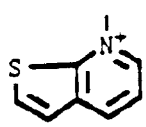 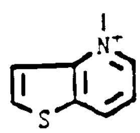 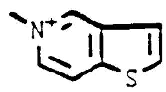 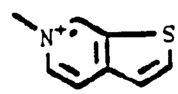 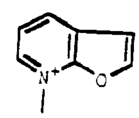 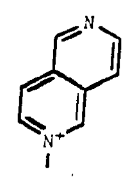

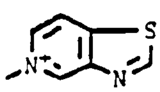 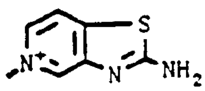 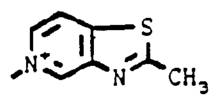 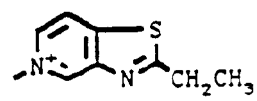 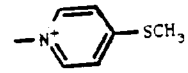

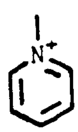 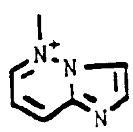 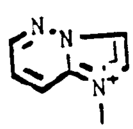 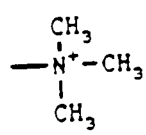 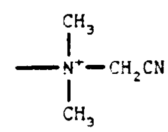

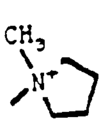 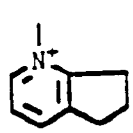 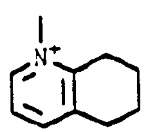 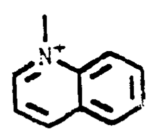 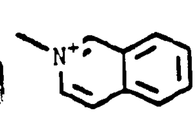 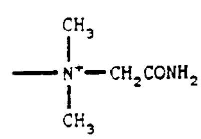

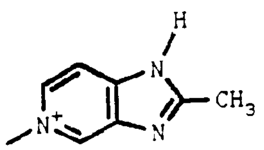 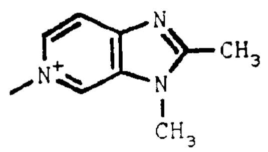 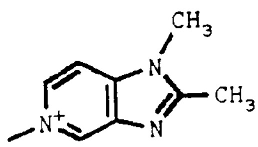 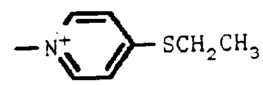

soit parmi les radicaux S-Het, dans lesquels Het représente les radicaux 1,2,4, 1,3,4 ou 1,2,3-thiadiazolyle ; thiazole-2-yle ; imidazol-2-yle ; 2-méthyl 1,3,4-thiadiazol-5-yle ; 1-méthyl tétrazol-5-yle et les radicaux :

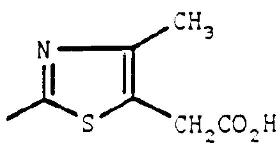 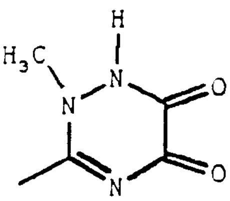 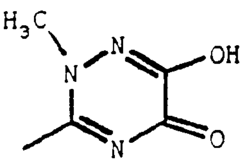

caractérisé en ce que soit l'on traite le produit de formule (IV) par un réactif capable d'introduire le radical $R_1$ tel que défini ci-dessus, soit l'on utilise au départ un produit de formule (VI) dans laquelle $R_1$ est tel que défini ci-dessus.

**3.**- Procédé selon la revendication 1 ou 2 pour préparer des produits de formule (I) telle que définie à la revendication 1 ou 2 dans laquelle $R_1$ est choisi
soit parmi les radicaux suivants :

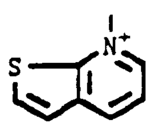 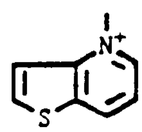 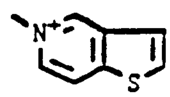 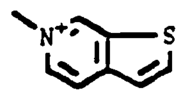

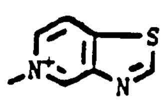 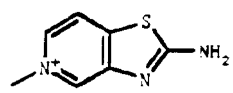 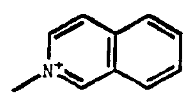

de préférence le radical :

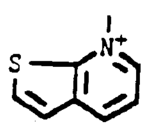

soit $R_1$ représente un radical 1,3,4-thiadiazolylthio ou méthyltétrazolylthio, caractérisé en ce que soit l'on traite le produit de formule (IV) par un réactif capable d'introduire le radical $R_1$ tel que défini ci-dessus, soit l'on utilise au départ un produit de formule (VI) dans laquelle $R_1$ est tel que défini ci-dessus.

**Revendications pour l'Etat contractant suivant : GR**

**1.**- Procédé pour préparer des produits de formule générale (I) :

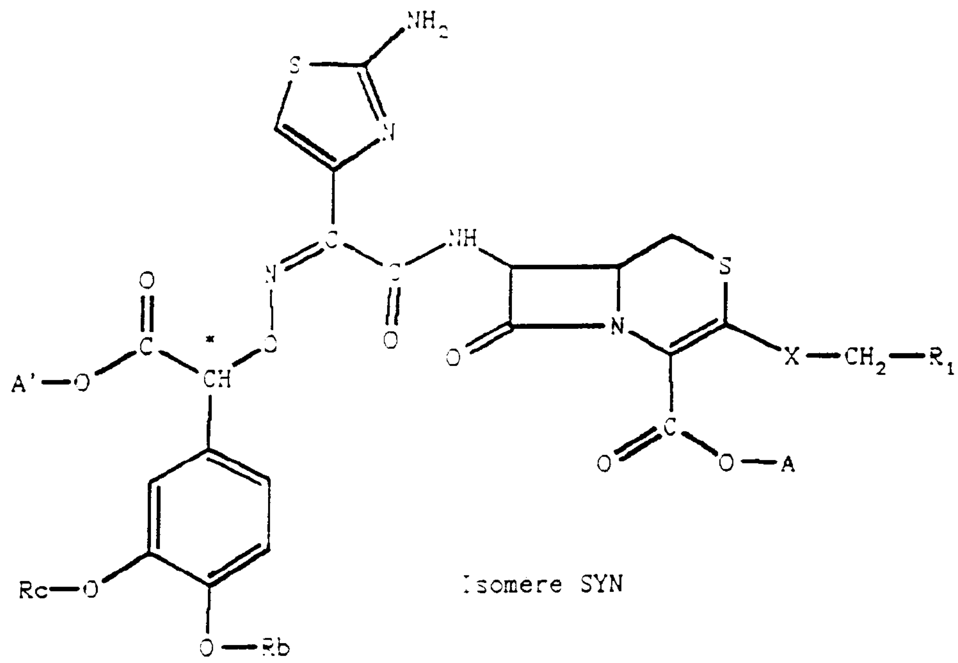

(I)

Isomere SYN

isomère syn, sous forme R ou S ou d'un mélange R, S, formule dans laquelle :
$R_1$ représente soit un radical choisi parmi les radicaux suivants :

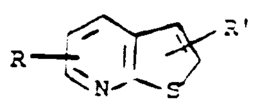
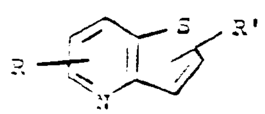
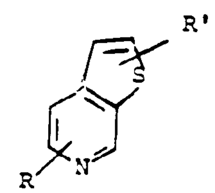
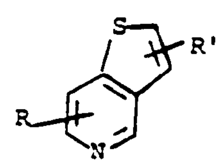

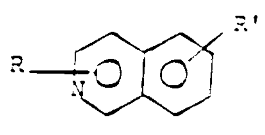
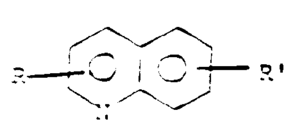
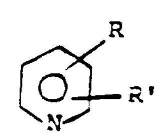
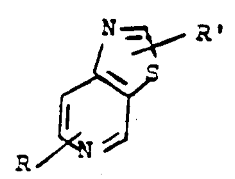

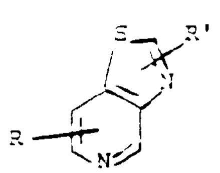
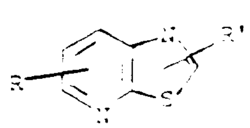
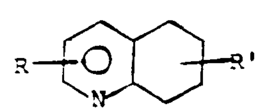
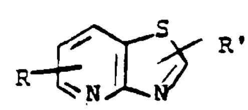

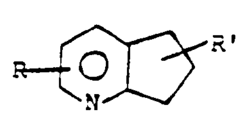
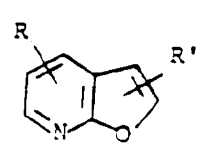
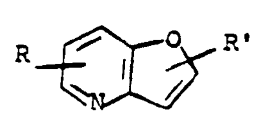
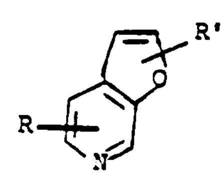

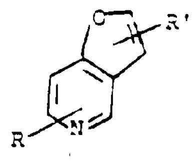
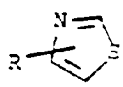
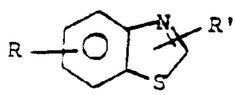
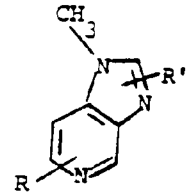
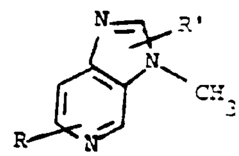

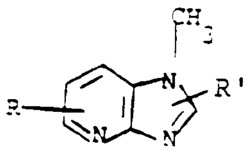
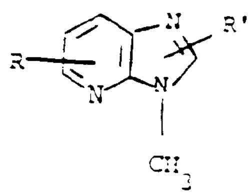
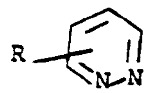
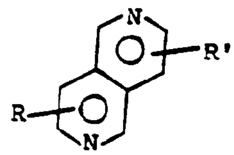

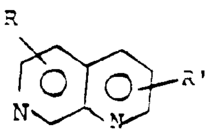
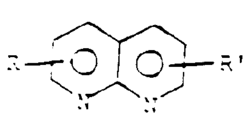
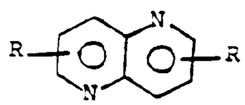
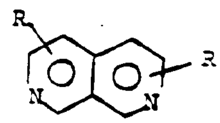

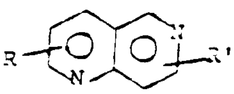
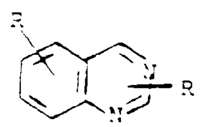
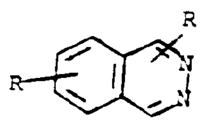
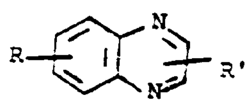

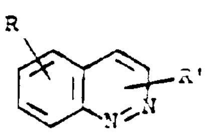
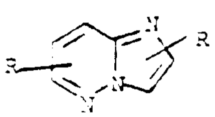
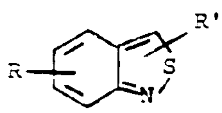
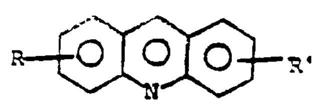

ou

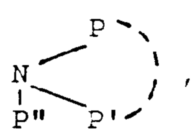

sous forme d'ammonium quaternaire, ou

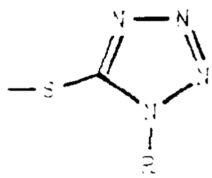

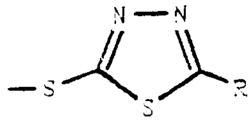

dans lesquels R et R′ identiques ou différents représentent un atome d'hydrogène, un radical alkyle renfermant de 1 à 4 atomes de carbone, un radical alcoxy renfermant de 1 à 4 atomes de carbone, un atome d'halogène, un radical $CO_2$-Q,

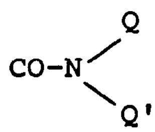 , 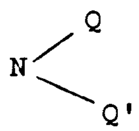 , 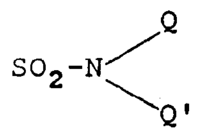 , 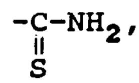

NH-CO-Q, CN, $CH_2$-CN, $CH_2$-SQ dans lesquels Q et Q′ identiques ou différents représentent un atome d'hydrogène ou un radical alkyle renfermant de 1 à 4 atomes de carbone, P, P′ et P″ identiques ou différents représentent un radical alkyle renfermant au plus 4 atomes de carbone, éventuellement substitué par un des substituants indiqués ci-dessus pour R et R′, le trait pointillé indiquant que P et P′ peuvent éventuellement former avec 5 l'atome d'azote auquel ils sont liés, un hétérocycle à 5 ou 6 chaînons,
soit $R_1$ représente un radical -S-Het dans lequel Het représente un hétérocycle choisi parmi les radicaux thiazolyle, isothiazolyle, oxazolyle, isoxazolyle, triazolyle, thiadiazolyle, oxadiazolyle, tétrazolyle, imidazolyle, pyrazolyle, pyridinyle, pyrimidinyle, pyrazinyle, pyridazinyle, triazinyle, dihydrotriazinyle, ces radicaux étant éventuellement substitués par un ou plusieurs substituants choisis parmi les radicaux alkyle ayant de 1 à 4 atomes de carbone, de préférence méthyle, amino, alkyl ou dialkylamino hydroxy, oxo, alkylthio, carboxy ou carboxyalkyle libres, estérifiés ou salifiés.
X représente une simple liaison ou un radical >C = C< sous forme E ou Z.
$R_b$ et $R_c$, identiques ou différents représentent un atome d'hydrogène ou un groupement acyle,
A et A′ identiques ou différents représentent un atome d'hydrogène, un équivalent de métal alcalin, alcalino-terreux, de magnésium, d'ammonium ou d'une base organique aminée ou A et A′ représentent le reste d'un groupement ester facilement clivable ou $CO_2A$ représente $CO_2^{\ominus}$ ainsi que les sels des produits de formule (I) avec les acides minéraux ou organiques, caractérisé en ce que
soit l'on fait agir un produit de formule (II) :

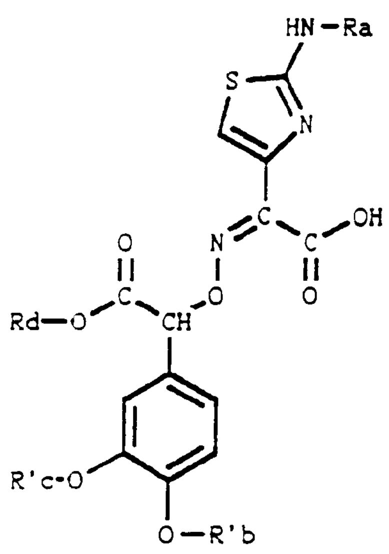

(II)

isomère syn, racémique ou optiquement actif ou un dérivé fonctionnel du produit de formule (II), dans laquelle

$R_a$ représente un atome d'hydrogène ou un groupement protecteur du radical amino, $R'_b$ et $R'_c$ identiques ou différents représentent un atome d'hydrogène ou un groupement protecteur du radical hydroxyle, $R_d$ représente un atome d'hydrogène ou le reste d'un groupement ester facilement éliminable, avec un produit de formule (III) :

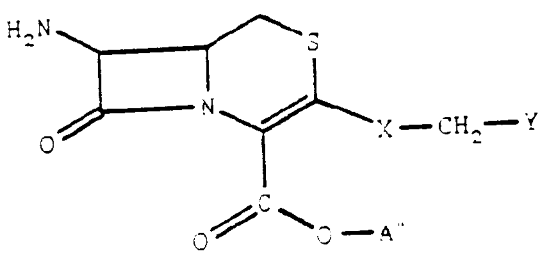

(III)

dans laquelle Y représente un radical hydroxy ou un atome d'halogène, A″ représente un atome d'hydrogène ou le reste d'un groupement ester facilement éliminable pour obtenir un produit de formule (IV) :

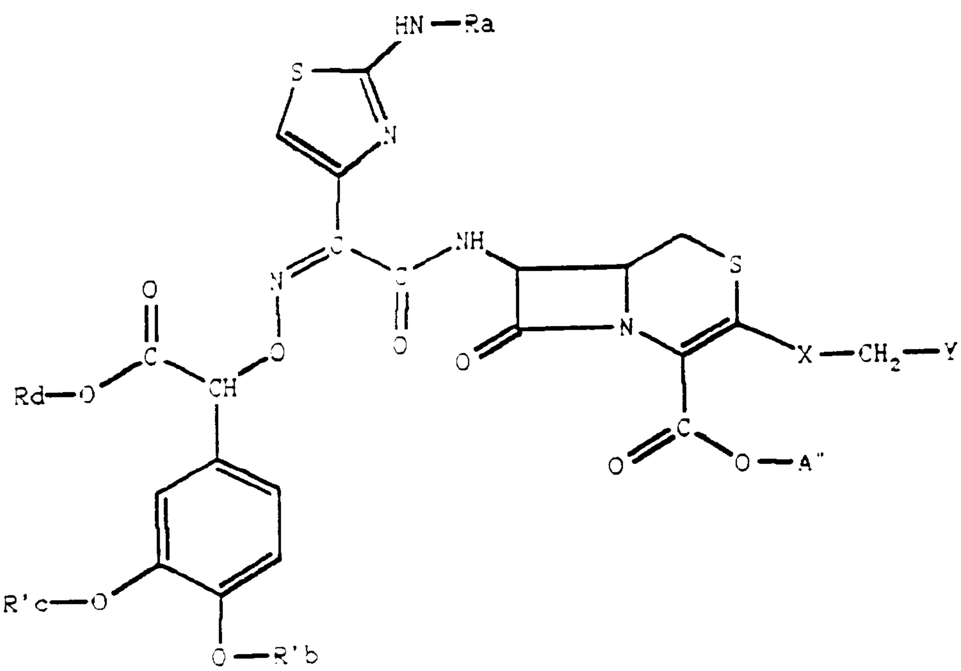

(IV)

que l'on fait réagir avec un réactif capable d'introduire le radical $R_1$ pour obtenir un produit de formule (V) :

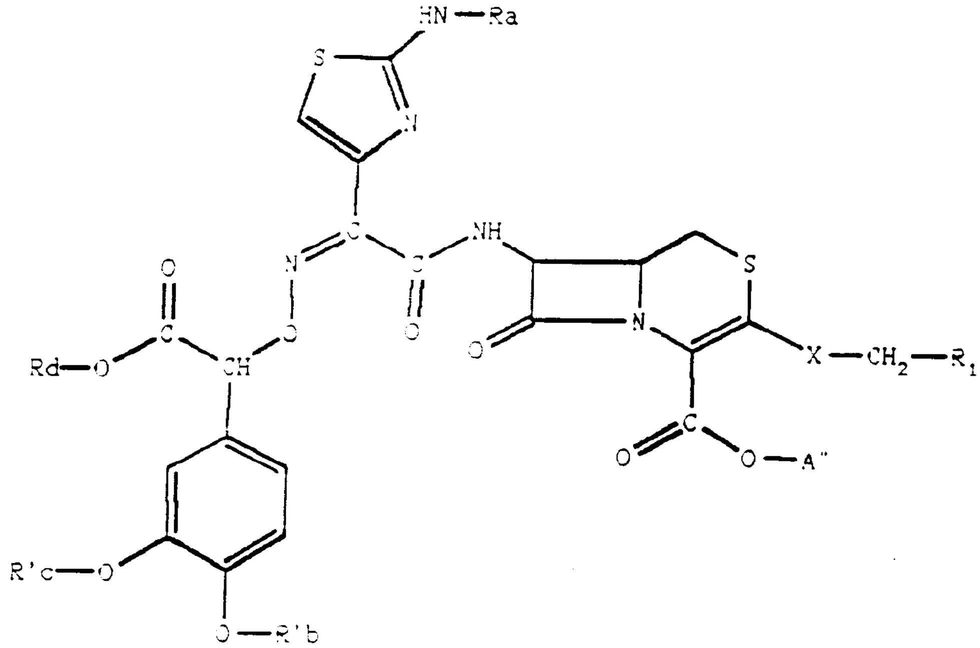

(V)

soit l'on fait réagir un produit de formule (VI) :

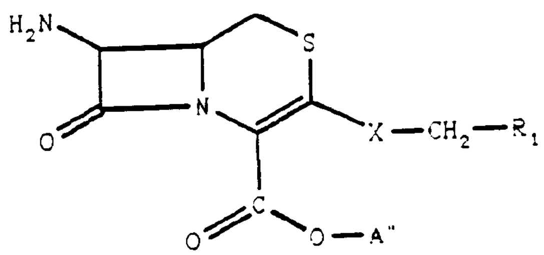

(VI)

dans laquelle X, $R_1$ et A″ ont la signification précédente avec un produit de formule (II) telle que définie ci-dessus ou un dérivé fonctionnel du produit de formule (II) pour obtenir un produit de formule (V) telle que définie ci-dessus,

produit de formule (V) que, si désiré, lorsque X représente un radical C = C l'on sépare en ses isomères E ou Z ou transforme les isomères Z en isomères E et produits de formule (V) que, si nécessaire ou si désiré, l'on soumet à une ou plusieurs des réactions suivantes, dans un ordre quelconque :

a) coupure par hydrolyse ou par action de la thiourée de tout ou partie des groupements esters ou des groupements de protection du radical amino ou des radicaux hydroxyles,

b) estérification ou salification par une base du ou des radicaux carboxyliques,

c) salification par un acide du radical amino,

d) séparation des produits sous forme de mélange R,S en R ou S.

**2.-** Procédé selon la revendication 1 pour préparer des produits de formule générale (I) telle que définie à la revendication 1, dans laquelle $R_1$ est choisi

soit parmi les radicaux :

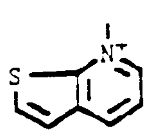 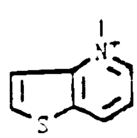 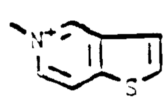 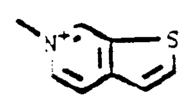 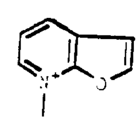 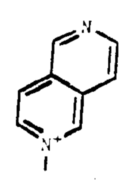

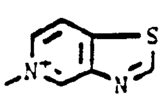 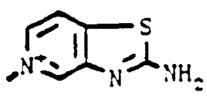 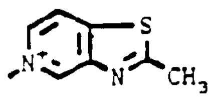 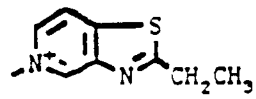 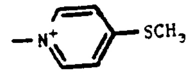

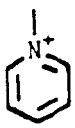 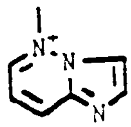 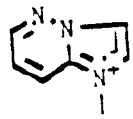 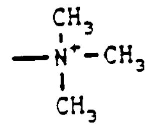 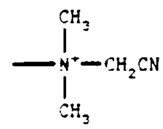

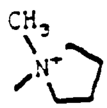 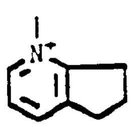 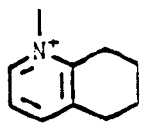 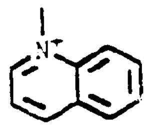 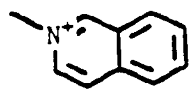 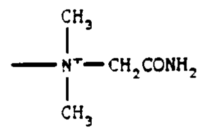

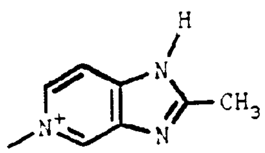 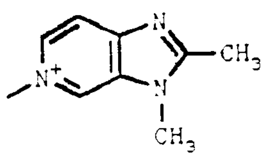 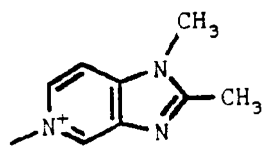 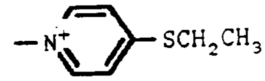

soit parmi les radicaux S-Het, dans lesquels Het représente les radicaux 1,2,4, 1,3,4 ou 1,2,3-thiadiazolyle ; thiazole-2-yle ; imidazol-2-yle ; 2-méthyl 1,3,4-thiadiazol-5-yle ; 1-méthyl tétrazol-5-yle et les radicaux :

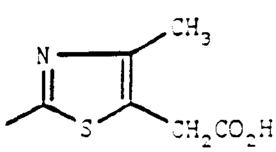 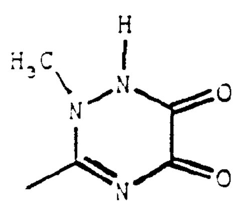 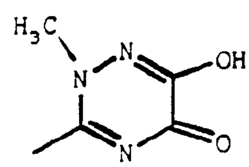

caractérisé en ce que soit l'on traite le produit de formule (IV) par un réactif capable d'introduire le radical $R_1$ tel que défini ci-dessus, soit l'on utilise au départ un produit de formule (VI) dans laquelle $R_1$ est tel que défini ci-dessus.

**3.-** Procédé selon la revendication 1 ou 2 pour préparer des produits de formule (I) telle que définie à la revendication 1 ou 2 dans laquelle $R_1$ est choisi
soit parmi les radicaux suivants :

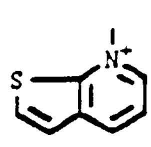 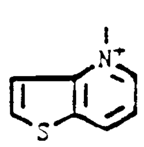 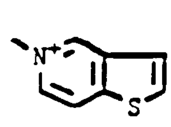 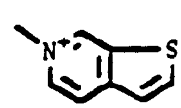

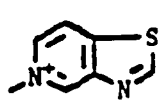 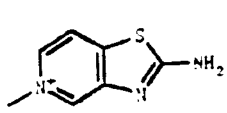 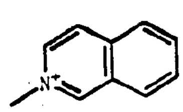

de préférence le radical :

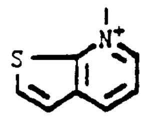

soit $R_1$ représente un radical 1,3,4-thiadiazolylthio ou méthyltétrazolylthio, caractérisé en ce que soit l'on traite le produit de formule (IV) par un réactif capable d'introduire le radical $R_1$ tel que défini ci-dessus, soit l'on utilise au départ un produit de formule (VI) dans laquelle $R_1$ est tel que défini ci-dessus.

**4.-** Procédé selon l'une quelconque des revendications 1 à 3, caractérisé en ce que l'on choisit les produits de départ et les réactifs de manière telle que l'on prépare le (S)(cis)(Z) 5-[[7-[[(2-amino 4-thiazolyl) [[carboxy-(3,4-dihydroxyphényl) méthoxy] imino] acétyl] amino] 2-carboxy 8-oxo 4-thia 1-aza bicyclo[4,2,0]oct-2-èn-3-yl] méthyl] thiéno[2,3-b]pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables.

**5.-** Procédé de préparation de compositions pharmaceutiques, caractérisé en ce que l'on met, à titre de principe actif, l'un au moins des produits de formule (I), telle que définie à la revendication 1, ou l'un au moins de ses sels avec les acides minéraux ou organiques, pharmaceutiquement acceptables, sous une forme destinée à l'usage thérapeutique.

**6.-** Procédé de préparation de compositions pharmaceutiques, caractérisé en ce que l'on met, à titre de principe actif, l'un au moins des produits de formule (I), telle que définie à l'une quelconque des revendications 2 à 4, ou l'un au moins de ses sels avec les acides minéraux ou organiques, pharmaceutiquement acceptables, sous une forme destinée à l'usage thérapeutique.

**7.-** A titre de produits industriels nouveaux, les produits de formule (IV) et les produits de formule (V) dans laquelle $R_a$ représente un groupement protecteur du radical amino, les formules (IV) et (V) étant telles que définies à la revendication 1.

Office européen des brevets

# RAPPORT DE RECHERCHE EUROPEENNE

Numero de la demande
EP 92 40 1765

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 153 229 (ROUSSEL-UCLAF) * revendications * | 1-9 | C07D513/04 A61K31/54 //(C07D513/04, 279:00,205:00) |
| Y | EP-A-0 214 029 (ROUSSEL-UCLAF) * page 114 - page 124; revendications * | 1-9 | |
| Y | EP-A-0 282 365 (ROUSSEL-UCLAF) * page 49 - page 54; revendications * | 1-9 | |
| Y | FR-A-2 586 019 (ROUSSEL-UCLAF) * page 118 - page 125; revendications * | 1-9 | |
| Y | FR-A-2 610 628 (ROUSSEL-UCLAF) * page 95 - page 98; revendications * | 1-9 | |
| Y | FR-A-2 610 629 (ROUSSEL-UCLAF) *Document complet.* | 1-9 | |
| Y | EP-A-0 359 291 (MOCHIDA) *Document complet.* | 1-9 | |

DOMAINES TECHNIQUES RECHERCHES (Int. Cl.5)

C07D

Le présent rapport a été établi pour toutes les revendications

| Lieu de la recherche | Date d'achèvement de la recherche | Examinateur |
|---|---|---|
| LA HAYE | 26 OCTOBRE 1992 | LUYTEN H.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)